(19) Europäisches Patentamt
European Patent Office
Office européen des brevets

(11) EP 2 188 394 B1

# (12) EUROPEAN PATENT SPECIFICATION

(45) Date of publication and mention of the grant of the patent:
**24.06.2015 Bulletin 2015/26**

(21) Application number: **08788296.5**

(22) Date of filing: **13.08.2008**

(51) Int Cl.:
***C12Q 1/68*** *(2006.01)*

(86) International application number:
**PCT/GB2008/002727**

(87) International publication number:
**WO 2009/022125 (19.02.2009 Gazette 2009/08)**

## (54) IDENTIFICATION OF NUCLEIC ACID SEQUENCES

IDENTIFIZIERUNG VON NUKLEINSÄURESEQUENZEN

IDENTIFICATION DE SÉQUENCES D'ACIDE NUCLÉIQUE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **13.08.2007 GB 0715739**
**13.08.2007 GB 0715737**

(43) Date of publication of application:
**26.05.2010 Bulletin 2010/21**

(73) Proprietor: **University of Strathclyde**
**Glasgow G1 1XQ (GB)**

(72) Inventors:
- **GRAHAM, Duncan**
  **Glasgow G1 1XL (GB)**
- **FAULDS, Karen**
  **Glasgow G1 1XL (GB)**
- **SMITH, Ewan**
  **Glasgow G1 1XL (GB)**
- **RICKETTS, Alastair**
  **Glasgow G1 1XL (GB)**

(74) Representative: **Chapman, Paul Gilmour et al**
**Marks & Clerk LLP**
**Atholl Exchange**
**6 Canning Street**
**Edinburgh EH3 8EG (GB)**

(56) References cited:

| | |
|---|---|
| **EP-A- 1 439 222** | **WO-A-99/44045** |
| **WO-A-03/078649** | **WO-A-2005/019812** |
| **US-B1- 6 268 146** | |

- **HILLIER S C ET AL: "An electrochemical gene detection assay utilising T7 exonuclease activity on complementary probe-target oligonucleotide sequences" ELECTROCHEMISTRY COMMUNICATION, ELSEVIER, AMSTERDAM, NL, vol. 6, no. 12, 1 December 2004 (2004-12-01), pages 1227-1232, XP004622962 ISSN: 1388-2481 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

This invention relates to the identification of nucleic acid sequences. More particularly the invention relates to the use of an exonuclease enzyme to facilitate the identification of a target sequence by way of degrading a nucleic acid duplex formed between a target nucleic acid sequence and a probe nucleic acid sequence, such that a discernable signal change is generated upon degradation of the probe when bound to/associated with the target or nucleic acid duplex, which signal change is detectable by way of Raman spectroscopy.

## Introduction

The modern molecular diagnostics industry is valued at £20 billion and is growing at a rate of 10% per year. Although reliant on a variety of different techniques, this ever-growing market is dominated by amplification-based approaches (mainly Polymerase Chain Reaction (PCR)-based assays). The reason for this is that the genome of organisms and indeed humans is particularly complex and requires simplification of the region of interest for a particular disease or diagnostic test. In addition to reducing the complexity of the genome, an amplification-based approach also increases the amount of material available for detection. This is important when considering the availability and sensitivity of routine techniques such as fluorescence or chemi-luminescence.

PCR is a molecular biological technique used to replicate and amplify specific regions (or genes) of a strand of DNA. A small amount of DNA can be amplified exponentially, without using a living organism such as yeast or E. *coli,* to give sufficient DNA to be adequately tested. The technique is used for a variety of applications such as the detection of infectious or hereditary diseases and disease states, the cloning of genes, paternity tests and genetic fingerprinting in forensics.

The PCR process is carried out in cycles. Each cycle consists of three steps: denaturation, annealing and elongation.

In the denaturation step the double-stranded DNA template that contains the target region or gene of interest that is to be amplified is heated to break the hydrogen bonds between the two strands. This causes them to separate and become accessible to primers (short sections of DNA or RNA that are complementary to the beginning and end of the region of DNA to be amplified).

In the annealing step the temperature is lowered, allowing the primers to hybridize to complementary sequences on the target DNA, flanking the region to be amplified. Since a large excess of the primers is often used, the target strands bind to the primers as opposed to each other.

In the elongation step DNA polymerase (an enzyme that synthesises new copies of the DNA region of interest) copies the DNA strand beginning at each primer and extends the new chains in the 5' to 3' direction. This results in two copies of the double-stranded DNA that make up the template for the next cycle. Thus, double the amount of DNA is replicated in each new cycle. A second cycle produces 4 copies of double-stranded target DNA sequence. After the third cycle there are 8 copies of the double-stranded target DNA sequence, two of which consist of just the target region. The other copies also include flanking DNA regions. Typically about 20 to 35 cycles are performed.

Quantitative PCR (QPCR), also referred to as real-time PCR, is a modification of PCR. The technique is commonly used to detect a specific sequence of DNA within a sample. If the specific sequence is present, QPCR can rapidly measure the quantity of product of PCR in real time. Thus it can be used to indirectly measure the amount of starting material present. Most QPCR methods use a fluorescent reporter molecule that increases as PCR product accumulates with each cycle of amplification.

One such technique is the SYBR Green method. The SYBR Green dye can bind the newly synthesised double-stranded DNA and the resulting increase in fluorescence intensity can be measured. This subsequently allows the initial DNA concentration to be determined.

Sequence-specific probes (e.g. TaqMan Probes or Molecular Beacons) are also commonly used for QPCR. TaqMan probes are designed to hybridise to a specific DNA sequence, usually a section of the desired PCR product. The probe contains a reporter dye that fluoresces. The probe also contains a quencher, which absorbs the fluorescence emitted by the reporter dye. The close proximity of the quencher to the reporter dye prevents fluorescence of the latter. During the elongation phase of the PCR cycle, the exonuclease activity of DNA polymerase allows it to 'overwrite' the probe breaking it into separate fragments. In doing so, the quencher molecule becomes separated from the reporter dye and fluorescence increases.

Molecular beacons act in a similar manner to TaqMan probes. Molecular beacons are hairpin-shaped probes that also typically contain a fluorophore/quencher pair and are designed to detect specific sequences of DNA. Again, the close proximity of the quencher prevents fluorescence of the fluorophore. When the probe hybridises to a complementary nucleotide sequence, however, the probe straightens out, introducing sufficient distance between the fluorophore and quencher for fluorescence to occur. Nevertheless, fluorescence detection is linked to sensitivity and a relatively large fluorescent signal may be required over background in order for successful detection to scan.

Radioactively labelled probes have also been used to detect DNA. Their use was advantageous in permitting very sensitive techniques but disadvantageous on account of difficulty in safe handling and disposal of the radiolabeled probes. In addition, such techniques did not allow for continuous (sometimes referred to as homogeneous) assays since separation of the unreacted substrate was required before any quantitative measurements could be made. Hence, such techniques are not used as frequently as more modern techniques such as those involving molecular beacons and TaqMan probes.

S.C. Hillier et al. report (Electrochemistry Communications, 6, 1227-1232 (2004)) an electrochemical gene detection assay utilising T7 exonuclease activity on complementary probe target oligonucleotide sequences in which a method is described for detecting a specific DNA sequence using a 5'- ferrocene labelled probe. The probe is hybridized to the target region and then the T7 exonuclease, which exhibits 5' to 3' exonuclease activity on double-stranded DNA, cleaves the terminal nucleotide at the 5' end of the probe releasing the ferrocene. The released ferrocene migrates to the electrode surface resulting in an increase in the ferrocene oxidation current at the electrode. However, this assay does not have any homogeneous aspects since any unincorporated or unhybridized probes would have to be separated prior to detection since these will otherwise give background signal.

US Patent No. 5,853,990 (Winger et al.) describes the determination of a nucleotide sequence by using an RNA probe. An RNA probe hybridizes to a target DNA sequence, the RNA probe having been modified so that it contains labels similar to that used in a TaqMan assay. The enzyme RNaseH is then used to selectively degrade the RNA strand of the chimera so as to release the label from the quencher.

## Summary of the Invention

The present invention is based on the recognition of the ability of certain exonucleases to digest double stranded (i.e. duplex) nucleic acid. Such a processing ability allows for a system to be provided whereby a signalling molecule can be released upon digestion of the nucleic acid duplex and the signalling molecule/label detected by way of Raman spectroscopy and in particular SE(R)RS.

Viewed from a first aspect, therefore, the invention provides a method for use in the detection of a target nucleic acid comprising the steps of:

(i) contacting a single-stranded probe nucleic acid with a sample of interest under conditions effective to generate a probe/target nucleic acid duplex by specific hybridisation of said probe nucleic acid to a target nucleic acid, if said target nucleic acid is present;
(ii) contacting any probe/target nucleic acid duplex with an exonuclease to effect digestion of the duplex and release of a label molecule from the duplex; and
(iii) detecting the label by Raman spectroscopy by detecting a detectable change in the Raman spectrum of the label upon its release from the duplex, so as to detect the target nucleic acid, if present.

Preferably, the exonuclease has no oligonucleotide-synthesising, or polymerase, ability; in other words contact with the exonuclease results only in oligonucleotide degradation, and not oligonucleotide construction.

The target nucleic acid is obtained from a sample of interest, which may be a fluid, liquid, air, swab from a solid surface etc.

In one embodiment of the invention the probe nucleic acid is labelled with the label. In another embodiment the label is able to bind to a duplex nucleic acid. The label is such that it may be detected by Raman spectroscopy, using methods such as surface-enhanced Raman scattering (SERS) or surface-enhanced resonance Raman scattering (SERRS) techniques. Suitable labels are disclosed in the prior art referred to hereinafter and may be referred to as "SE(R)RS labels" which term refers to a label which may be detected by SERS and/or SERRS.

A particular advantage of the present invention, in contrast to the prior art, such as PCR, is that amplification of the target nucleic acid need not necessarily be effected, because a detectable signal, in particular with SE(R)RS, provides sufficient sensitivity with minute quantities of target nucleotide.

The use of SE(R)RS as a detection modality is disclosed, for example, in WO97/05280, WO99/60157 and WO2005/019812 as well as in numerous research papers, in particular those (co)authored by Duncan Graham (see for example SERRS Dyes. Part 2. Synthesis and evaluation of dyes for multiple labelling for SERRS (McHugh, C.J., Docherty, F.T., Graham, D., Smith, W.E. Analyst, 2004, 129, 1, 69-72); and Biosensing Using Silver Nanoparticles and Surface Enhanced Resonance Raman Scattering (Graham, D, Faulds, K, Smith, W. E., Chemical Communications, 2006, 42, 4363-4371)).

In WO97/05280, WO99/60157 and WO2005/019812 the use of SE(R)RS in methods for detecting or identifying particular nucleic acid sequences is described. In these publications detection is based upon/measurement of the direct effect of binding of a target sequence on SE(R)RS. In contradistinction, it is to be appreciated that the present invention permits the detection of a target sequence by virtue of the detectable release of a label by virtue of digestion of a nucleic

acid duplex, which release is indicative of the probe having been bound to the target sequence.

The combination of Raman or SE(R)RS detection with nucleic acid sequence identification in which an exonuclease is used to release a Raman/SE(R)RS detectable label from a hybridised probe/target duplex has not, to the best of our knowledge, hitherto been reported and represents a further aspect of the invention. Viewed from this aspect the invention provides a method for use in the detection of a target nucleic acid comprising the steps of:

(i) contacting a single-stranded probe nucleic acid with a sample of interest under conditions effective to generate a probe/ target nucleic acid duplex by specific hybridisation of said probe nucleic acid to a target nucleic acid, if said target nucleic acid is present;
(ii) contacting any probe/target nucleic acid duplex with an exonuclease to effect digestion of the duplex and release of a label molecule from the duplex; and
(iii) detecting the label by Raman spectroscopy by detecting a detectable change in the Raman spectrum of the label upon its release from the duplex, so as to detect the target nucleic acid, if present.

Viewed from a still further aspect the invention provides a method for simultaneously detecting a plurality of different target nucleic acids in a sample of interest comprising simultaneously effecting a plurality of methods according to the invention for use in the detection of a target nucleic acid in which a different label is used for detecting each of said target nucleic acids.

Also disclosed herein is a kit of parts comprising:

(i) a probe nucleic acid;
(ii) an exonuclease capable of digesting double stranded nucleic acid; and
(iii) a SE(R)RS detectable label

In certain embodiments, the probe nucleic acid in the kit is a single-stranded probe nucleic acid having a 5-phosphate group; and the exonuclease is lambda exonuclease.

The ability to detect sometimes small quantities of target nucleic acids in the absence of replication of the target is a particular advantage of the present.

Further disclosed is a method for use in the detection of a target nucleic acid comprising the steps of:

(i) contacting a single-stranded probe nucleic acid with a sample of interest under conditions effective to generate a probe/target nucleic acid duplex by specific hybridisation of said probe nucleic acid to a target nucleic acid, if present; and
(ii) contacting any probe/target nucleic acid duplex with an exonuclease to effect digestion of the duplex and release of a label molecule from the duplex,

wherein an excess of said probe nucleic acid is contacted with the sample of interest in step (i) such that digestion of a duplex in step (ii) recycles the target nucleic acid one or more times thereby allowing further probe molecules to specifically hybridise to the target forming additional probe/target nucleic acid duplexes that are digested to release further label molecules.

Also disclosed is the use of a target nucleic acid as a template with which a single-stranded probe nucleic acid is specifically hybridised and a resultant probe/target nucleic acid duplex degraded by an exonuclease to release a label molecule from the duplex, wherein said target nucleic acid is used as said template a plurality of times so as to release a plurality of label molecules from a plurality of duplexes, and wherein each of said plurality of duplexes is comprised of the same target nucleic acid.

**Brief description of the drawings**

Figure 1(a) shows schematically an embodiment of the present invention making use of SERRS detection. Initially a sample containing genomic, unamplified DNA is taken and heated to render the DNA single-stranded. A probe is then added which binds specifically to the target sequence of DNA. This probe is designed in a specific manner depending on the detection strategy to be used: it may have a 5' phosphate group for example for it to allow the action of $\lambda$ exonuclease enzyme to occur. In the case of SERRS detection the probe may, for example, contain a 3' hydroxyquinoline dye which does not give a SERRS signal whilst attached to the probe. Upon action of the enzyme and digestion of the probe, however, the dye is released into solution allowing a SERRS signal to be obtained on the addition of a suitable substrate, such as silver nanoparticles.

Figure 2 shows a further embodiment of the present invention wherein the detection probe is a metal nanoparticle attached to the surface of which are the probe sequences. These stop the nanoparticles from aggregating due to electrostatic repulsion. The probe sequences may be modified in different ways and in particular to facilitate SERRS detection as well as visual detection. System (a) uses probes attached to the surface at the 3' end and which have been modified at the 5' end with a phosphate group. Upon hybridisation to the target sequence and subsequent digestion by an exonuclease, the DNA is degraded resulting in aggregation of the particles, which can be observed as a shift in the surface plasmon and optionally a distinctive colour change. In system (b) SERRS active dyes will be spaced on the surface of the nanoparticles, however no signal will be observed until after hybridisation to the target and digestion by the enzyme and the resulting aggregation occurs. After aggregation has occurred a SERRS signal will be obtained. In system (c), the probe used is the same probe used in Figure 1; upon hybridisation and digestion the SERRS dye will be released and be able to attached to the surface and give a SERRS signal when the nanoparticles hybridise.

Figure 3 is a schematic flow diagram of an assay employing λ exonuclease to digest an oligonucleotide probe when bound to target nucleic acid and removal of undigested probe.

Figure 4 shows the increase in fluorescence over time when double-stranded DNA in which one strand bears a 5'-phosphate moiety is exposed to λ exonuclease, and a comparison with the same experiment conducted in the absence of λ exonuclease.

Figure 5 shows the increase in fluorescence over time when double-stranded DNA in which one strand bears a 5'-phosphate moiety is exposed to two different amounts of λ exonuclease, and a comparison with the same experiment conducted in the absence of λ exonuclease.

Figure 6 shows increases in fluorescence upon processing by λ exonuclease upon the same double-stranded DNA exposed to λ exonuclease the results of which are depicted in Figures 1 and 2 and modified DNAs in which the substrates have a 5' recess; a blunt 5' end and a 3' tail; a 5' recess and a 3' tail; and a comparison with unmodified DNA is the absence of λ exonuclease.

Figure 7 shows SERRS spectra of assay reactions after enzyme digestion and the subsequent clean-up step in the presence/absence of the complementary sequence.

Figure 8 shows SERRS intensity of the average main peak height at 1650 $cm^{-1}$ of the TAMRA-labelled probe (PTBPROBE) in the presence/absence of the complementary target sequence after being digested by λ exonuclease enzyme. Control reactions run under the same condition but lacking the enzyme are also shown for comparison. The value quoted is the average of three measurements carried out for 3 separate replicates for each type of reaction.

Figure 9 shows SERRS spectra of the assay reaction after digestion, inactivation and biotin removal step (dotted line) and the same reaction carried out in the absence of the enzyme (straight line).

Figure 10 shows a schematic representation of a FRET-based assay.

Figure 11 shows an assay format used in low target concentration catalysis studies. The dye-labelled probe is shown as an 8-pointed star, the minor groove binder as a cross (X) and the target as a thick line. Different ratios of FAM-labelled probe to complementary target sequence (1:1; 10:1, 100:1) were digested in the presence of lambda exonuclease enzyme. The concentration of FAM-labelled probe and H33258 was kept constant at 1 μM; the concentration of complementary sequence was reduced. The reaction mixture was heated to 37 ºC for 30 mins followed by an enzyme inactivation step at 75 ºC for 15 mins. As the level of fluorescence was below that of the detector an excess of target was added to attempt duplex formation and hence produce a melting curve if there was appreciable amounts of probe left undigested.

Figure 12 shows the change in fluorescence intensity over 30 mins of the FRET duplex in the presence (lower curve)/absence (upper curve) of lambda exonuclease. A decrease in fluorescence intensity indicates the destruction of the FRET system by digestion. Ratio of labelled probe: complement (1:1).

Figure 13 shows fluorescence annealing curves for samples containing an initial excess of the probe in comparison to the target sequence (100:1). Both samples were spiked with extra target. The lower trace shows sample in which enzyme was added, showing no major change in fluorescence intensity. The upper trace lacked the enzyme and

hence shows a sharp fluorescence transition -58 C, which correlates to the Tm of the duplex for this sequence.

Figure 14 shows fluorescence of control single-stranded FAM probe in the presence of H33258 and λexo (lower solid line); double-stranded DNA (hybridised FAM probe) with H33258 (upper solid line); and double-stranded DNA with H33258 and λexo (dotted line), showing the progress of the digestion within 30 minutes.

Figure 15 shows annealing curves for undigested (solid line) and digested (dotted line) DNA. Undigested sample shows a Tm = 57ºC.

Figure 16 shows change in intensity after 30 min digestion with λexo using different complements for FAM probe. For every pair, the left-hand side bar represents change in fluorescence of samples without enzyme, right-hand side samples with enzyme. (showing averages of three measurements except from 5' overhang, only one measurement).

Figure 17 shows a schematic representation of a method in which a single-stranded probe nucleic acid comprising a SE(R)RS label and biotin is used, unreacted or excess probe being removed using streptavadin-coated magnets allowing detection of only SE(R)RS label that was present in a probe/target duplex prior to degradation by the exonuclease.

Figure 18 shows an agarose TBE gel showing detection of target nucleic acid (C. *trachomatis* DNA) (lanes 2 to 9) versus a negative control (lanes 12 to 19).

Figure 19 depicts the results of SERRS analysis of C. *trachomatis* DNA with negative controls, and with varying probe concentration.

Figure 20 depicts SERRS spectra of C. *trachomatis* DNA containing samples, versus a negative control.

Figure 21 depicts, schematically an assay protocol in which target nucleic acid is contacted, sequentially with a biotinylated capture probe bound to streptavadin-coated magnetic beads and then a SERRS active dye-labelled 5'-phosphate-terminated probe, the resultant duplex being digested by lambda exonuclease, allowing detection of the dye using SERRS.

Figure 22 shows a SERRS response of an assay depicted in Figure 21 (upper spectrum) with a negative control in which no dye-labelled probe is used (lower spectrum).

Figure 23 shows a SERRS spectrum of an 8-hydroxyqunoline-derived dye.

Figure 24 shows a method of the invention involving the release of a label from a probe/target nucleic acid duplex formed upon hybridisation of two probe nucleic acids and a target nucleic acid.

Figure 25 shows a variation of the embodiment depicted in Figure 24 in which a target DNA is captured by a capture probe secured to a solid surface such as a bead or a plate.

**Detailed description of the Invention**

The terms "target", "sample" or "sample of interest" refer herein to, any nucleic acid-containing sample e.g. nucleic acid-containing samples isolated from (an) individual(s). The "target nucleic acid" can be any nucleic acid, including DNA (from any source e.g. genomic, cDNA, synthetic etc.), RNA (e.g. mRNA, tRNA, rRNA, synthetic etc.) or derivatives (such as the inclusion of rare/unusual naturally derived nucleotide bases and/or synthetic nucleotide bases, known in the art), of these. The sample can represent all or only some of the nucleic acid present in a given source. The sample may be prepared prior to testing in order to make the target nucleic acid therein more available for the testing process. For instance the target nucleic acid may be fully or partially purified and/or fragments may be produced and separated. As an alternative to, or in addition to, using the nucleic acid in the sample directly, copies may be prepared and used (e.g. by PCR). The term "target nucleic acid" covers all of these possibilities.

The terms "nucleic acid", "polynucleotide" and "oligonucleotide" are used herein to a polymer, or oligomer based upon nucleotides containing 2-deoxy-D-ribose or D-ribose, as well as PNA or LNA molecules known in the art. Thus, these terms include dsDNA and ssDNA as well as dsRNA and ssRNA. The terms also embrace the possibility of chimeric mixtures of the foregoing, an example of such a mixture being a single-stranded oligonucleotide construct comprising both DNA and PNA or LNA. There is no intended distinction between the terms "nucleic acid", "polynucleotide" and

"oligonucleotide"; these terms are used interchangeably.

The target nucleic acid need not be a species isolated from any existing or natural sequence but may be any sequence of any length present in, or within, the sample which it is desired to investigate. Thus it may be any sequence found in a genome, or subgenomic nucleic acid, chromosome, extrachromasomal vector, or gene, or motif, or non-coding sequence, or a sequence tagged site, or expressed sequence tag. The sequence may be derived from any source e.g. made according to published material or that on a database.

The term "specific hybridisation" is intended to refer to the probe nucleic acid sequence being substantially complementary to that of the nucleic acid sequence of the target. This refers to the oligonucleotides which, when aligned such that the 5' end of one sequence is paired with the 3' end of the other, there is at least 95%, typically at least 97%, more typically at least 98% and most typically at least 99% identity (i.e. Watson-Crick base-pairing) between the sequences. Modified base analogues not commonly found in natural nucleic acids may be incorporated (enzymatically or synthetically) in the nucleic acids. As is known in the art complementarity of two nucleic acid strands may not be perfect: some stable duplexes may contain mismatched base pairs or unmatched bases and one skilled in the art of nucleic acid technology can determine their stability hypothetically by considering a number of variables other than the length of the oligonucleotide and concentration and identity of cytosine and guanine bases in the oligonucleotide. In this regard it will be understood that the stringency of the solutions used in any given case can be varied according to the requirements of the example concerned, and selection of the appropriate stringency is within the capability of the skilled person.

The skilled addressee is aware that appropriate control of temperature and/or salt concentrations, for example, it is possible to ensure that only probe sequences of a desired degree of specificity for a particular target remain hybridised to the target and non-specific probes are not hybridised to the target. The skilled addressee understands this to relate to specific hybridisation.

As is known in the art, the parameters of salt concentration and/or temperature can be varied to achieve the desired identity between the probe and target nucleic acid. Guidance regarding such conditions is readily available to those skilled in the art and in particular may be found in Sambrook et al., Molecular Cloning: A Laboratory Manual (Third Ed.), Cold Spring Harbor Laboratory Press, Cold Spring Harbor, NY, pages 7.9 to 7.12 (2001).

The stability of a nucleic acid duplex is measured by the melting or dissociation temperature, or "Tm". The Tm of a particular nucleic acid duplex under specified reaction conditions is the temperature at which half of the base pairs are present in a duplex structure and half are present in single-stranded DNA.

The principal governing factors determining Tm are sequence length and G-C content. The theoretical and experimental procedure for determining the Tm is disclosed in Molecular Cloning-A Laboratory Manual, Second Edition, J Sambrook et al., Cold Spring Harbor, Chapter II section 46 and 55. In essence, for oligonucleotides shorter than 18 nucleotides, the Tm of the hybrid is estimated by multiplying the number of A + T residues in the hybrid by 2 °C and the number of G + C residues by 4°C and adding the two together. For oligonucleotides between approximately 14 and 70 nucleotides in length, the following equation devised by Bolton and McCarthy, (P.N.A.S. 48:1390, 1962) for determining TM of long DNA molecules is also applicable:

$$Tm = 81.5 - 16.6(\log[Na+]) + 0.41(\%\ G + C) - (600/N).$$

(wherein N chain length and [Na+] is the ionic strength of the hybridisation solution).

Other formulae for calculating Tms are known to those skilled in the art.

As noted above, the skilled person is aware that the exact Tm will depend on many factors, such as the reaction temperature, salt concentration, the presence of denaturants such as formamide, and the degree of complementarity with the sequence to which the oligonucleotide is intended to hybridise.

With oligonucleotide hybridisation, the optimum hybridisation temperature is generally carried out under conditions that are 2 to 10 °C below the Tm. Ideally, the hybridisation temperature is controlled precisely, preferably to $\pm 2$°C, more preferably to $\pm 0.5$°C or better, particularly when the hybridisable length of the capture oligonucleotides are small and there is a need to discriminate between two sequences that may only differ by a single nucleotide at one or other of the termini of the hybridisable sequence.

Hybridisation conditions chosen are designed to be as close as possible to the calculated Tm of the duplexes formed between the probe and target nucleic acids. The concentration of salt in the hybridisation solution used is particularly significant. At 1 M NaCl, G:C base pairs are more stable than A:T base pairs. Similarly, double stranded oligonucleotides with a higher G-C content have a higher Tm, than those of the same length but with a higher A-T content. If slight differences, i.e. single nucleotide differences, amongst the target nucleic acids need to be distinguished, establishing optimum hybridisation conditions is important, particularly, when the hybridisable length of the oligonucleotides is small (< approximately 30-mers). Where, because of a diverse composition of nucleotides in the sample of interest in addition to the target nucleotide, there is a broad range of Tms. Because of this conditions may be manipulated so

as to diminish the Tm's dependence on nucleotide composition by using chaotropic hybridisation solutions. This can be effected, for example, by incorporation into the hybridisation solution of a tertiary or quaternary amide.

As used herein, the probe nucleic acid or "probe" comprises a nucleic acid sequence which is designed to form a duplex structure with a substantially complementary sequence in the target nucleic acid. It is to be understood that the probe nucleic acid may thus form a duplex, for example, with a single-stranded or double-stranded target nucleic acid. With the latter the resultant triplex structure will comprise a duplex formed by hybridisation between one strand of the target nucleic acid and the single-stranded probe. Those skilled in the art will understand that higher order duplex-containing oligonucleotide architectures are possible, for example quartet structures.

In certain embodiments, the label is directly attached, bonded or otherwise associated with one or more nucleotide bases of the "probe" nucleic acid. By associated with is to be understood to embrace embodiments in which an individual nucleotide, or part thereof, is detectable once no longer attached to the original probe nucleic acid of which it forms part.

Suitable labels for detection by Raman/SE(R)RS are described in, for example, WO97/05280, WO99/60157 and WO2005/019812. Other suitable labels include those dyes disclosed in SERRS Dyes. Part 2. Synthesis and evaluation of dyes for multiple labelling for SERRS (McHugh, C.J., Docherty, F.T., Graham, D., Smith, W.E. Analyst, 2004, 129, 1, 69-72); and Biosensing Using Silver Nanoparticles and Surface Enhanced Resonance Raman Scattering (Graham, D, Faulds, K, Smith, W. E. Chemical Communications, 2006, 42, 4363-4371). It is also possible that the label has a particular resonance frequency, which frequency can change when a number of the labels are brought into close proximity.

Fluorescent labels are commonly used in various detection systems and a variety of fluorescent labels are known to those skilled in the art. The present invention may also employ the techniques of FRET and/or quenching of fluorescent signal, as will be described in more detail, hereinafter.

Thus the term "label" refers to any atom or molecule that can be used to provide a detectable, preferably quantifiable, preferably real-time, signal. The detectable label can be attached to or is inherently part of the nucleic acid probe or may become otherwise bound to the duplex formed between the probe nucleic acid and the target nucleic acid. Such labels may bind to the minor or major groove of the duplex, or otherwise intercalate with duplex nucleic acid. It is understood however, that labels of this sort should not be capable of binding to the target nucleic acid unless a duplex is formed between the probe and target nucleic acid. Many such labels are known and include PicoGreen® (G. Tolun and S. Myers (A real-time DNase assay (ReDA) based on PicoGreen® fluorescence Nucleic Acids Res. 2003, 31: e111)).

As used in this application, "real time" refers to detection of the kinetic production of signal, comprising taking a plurality of readings in order to characterize the signal over a period of time. For example, a real time measurement can comprise the determination of the rate of increase of detectable product. Alternatively, a real time measurement may comprise the determination of time required before the target sequence has been amplified to a detectable level.

In some embodiments it may be necessary to remove unhybridized probe from a reaction, in order to allow detection of only label which has become incorporated/ associated with a probe/target nucleic acid duplex. One way of achieving this is to incorporate a capturing moiety (also referred to herein as a capturable moiety) on/to the probe nucleic acid (examples include a magnetic moiety and/or biotin). If the probe nucleic acid does not bind to the target, the probe will not be digested by the exonuclease and the undigested probe can be removed by a magnet such as magnetic beads, in the case of a magnetic moiety or using a streptavadin-coated material, in the case of biotin. However, if the probe forms the probe/target duplex, the capturing moiety will be released from the probe upon duplex digestion but the label can still be detected.

Thus for example, by dual labelling a probe with a label detectable by Raman spectroscopy (e.g. SE(R)RS) and biotin, e.g. at the 3'-end, undigested probe (e.g. because the target nucleic acid was not present) or excess probe can be removed by use of streptavadin-coated magnetic beads, meaning that a signal is only detected when the target nucleic acid is present.

This is shown schematically in Figure 17 (using SE(R)RS detection and biotin/ streptavadin capture as an example).

This methodology may be understood to be analogous to the use of Taq-Man probes. With Taq-Man probes the signal is suppressed by the use of a quencher which, once this is separated from the reporter dye during amplification in PCR, no longer suppresses the fluorescence of the reporter dye and fluorescence is detectable. In the present invention, however, use of the quencher may be obviated, since signal which would otherwise arise as a consequence of excess or unhybridised probe can be removed by the use of a capturable moiety such as biotin being attached to the probe as well as a Raman-detectable dye. Advantageously, minimal probe may be used where the sensitivity of the detection modality permits (e.g. when using SE(R)RS).

In still further embodiments of the invention, methods of the invention can optionally make use of more than one probe nucleic acid acting in combination to probe for the target nucleic acid. Thus, for example, use of two probes, one serving to capture the target nucleic acid, if present in the sample of interest and the other to provide a (typically) detectable label to signal that the target is present, may be deployed. Thus a first probe nucleic acid may be, for example,

biotinylated at the 3'-end, whereby to provide a capturable moiety. This may be contacted with a sample of interest. If the target nucleic acid is present, this hybridises to the first probe, and the resultant duplex captured using streptavadin-coated magnetic beads. Unbound probe and other components in the sample of interest may then be washed away. Alternatively the first probe nucleic acid may be initially contacted with a streptavadin-coated magnet (or magnetic beads), unbound capture probe being washed away before exposure to the sample of interest, after which unhybridised components of the sample of interest may be washed away. This alternative embodiment is depicted schematically in Figure 21.

After capture of the target nucleic acid, a Raman-detectable label may then be introduced to the duplex resultant from hybridisation of the first probe nucleic acid to the target nucleic acid. This can be achieved by using a label that can intercalate with the duplex as described herein. Alternatively, and typically the label is introduced with a second probe nucleic acid which is hybridised to the target nucleic acid already bound to the first probe nucleic acid. The resultant duplex comprising strands of first and optionally second probe nucleic acids complementary to different regions of the target nucleic acid may then be degraded by the exonuclease to liberate the Raman-detectable (e.g. SE(R)RS active) label.

In all embodiments of the invention, where it is possible to separate unbound label from the target nucleic acid, e.g. by using the capture technique described herein, it is possible and is often advantageous to not include a quenching moiety for the Raman-detectable label in the probe where this comprises the label. This is because it is possible to remove unbound label-containing probe (which might otherwise give rise to a background false positive result, given that the target nucleic acid is indicated by the detection of label). However, where the capture techniques described herein are not used, or even where they are used, it can be advantageous to include a quencher for the label in a probe nucleic acid. In this way any residual, but unhybridised label-containing probe does not contribute to any signal detected.

Figure 21 depicts, schematically, an example of an embodiment described hereinbefore in general terms in which separate first and second nucleic acid probes are employed with a particular, but not obligatory, series of washing stages depicted. It will be observed that the second probe nucleic acid comprises a label and a quencher (the quencher being the darker circle in the "label" probe) although it will be understood from the immediately preceding discussion that the presence of this quencher is not an essential feature. The "P" depicts a terminal 5'-phosphate moiety serving to render the resultant double-stranded nucleic acid as a substrate for lambda exonuclease. Thus Figure 21 shows a short capture probe (3' biotinylated) bound to streptavadin-coated magnetic beads. Unbound probe is washed away. The target sequence is then hybridised to the immobilised capture probe (first probe nucleic acid). Unbound target nucleic acid is washed away. A dye-labelled oligonucleotide sequence (with a 5' phosphate group) is then hybridised to captured target sequence. The action of $\lambda$ exonuclease occurs on the hybridised probe sequence. The enzyme degrades the hybridised probe DNA to mononucleotides, freeing the dye.

As an additional example of an embodiment of the invention that can make use of more than one nucleic acid acting in combination to probe for the target nucleic acid, there can be used two probe nucleic acids in which a first probe comprises a sequence designed to probe for the target nucleic acid. In addition to the portion of the first probe that is complementary to at least a portion of the target nucleic acid the first probe also comprises a sequence complementary to a second probe nucleic acid. This second probe nucleic acid may comprise a SE(R)RS or other Raman active label (that is at least SE(R)RS or otherwise Raman detectable when cleaved from the second probe nucleic acid) and optionally a quencher. Alternatively, the label can be provided otherwise, e.g. being a label that can intercalate into a duplex formed by the hybridisation between the target nucleic acid and the probe nucleic acid (constituted in this embodiment by first and second probe nucleic acids) as described herein. The first or second probe, typically the second probe, may also comprise a 5'-phosphate group where the exonuclease used is lambda exonuclease. Figure 24 shows a schematic depiction of this embodiment of the invention in which the detection modality is indicated to be SERRS; the target nucleic acid a DNA; the second probe nucleic acid labelled with a SERRS active dye (shown as a star) and having a 5'-phosphate; and the exonuclease used being lambda exonuclease.

One advantage of this embodiment of the invention is that it allows the first probe to function as a target-specific probe based upon that portion of its sequence designed to hybridise to the target nucleic acid. The portion of its sequence description to hybridise to the second probe may be generic, by which it is meant that the sequence is not specific to the target nucleic acid but rather to the second probe. The second probe is thus not required to possess any sequence complementarily for the target nucleic acid and may thus be used to detect multiple target nucleic acids. This is advantageous because the second probe nucleic acid, as is indicated, is typically more structurally complex, typically comprising a label and optionally a quencher and optionally a 5'-phosphate group. The second probe need not be custom-made based upon the target nucleic acid; the first probe, which is based in part upon a sequence found in the target nucleic acid, may be made by simple oligonucleotide synthesis.

This approach can allow a generic probe to be used for the assay i.e. the sequence of the second probe (and its complementary sequence in the first probe) can be used for every target nucleic acid and can be optimised to give the best possible conditions for the enzyme to work, the hybridisation to occur and for the optimum label configuration.

The target region of the first probe nucleic acid can then be changed to hybridise to the target. Particularly

advantageously this portion of its sequence could also contain bases which will increase the hybridisation efficiency such as LNA bases.

A further advantage of this embodiment of the invention is that it allows straightward detection of target nucleic acids that comprise PNA or LNA: since it is not the (optionally chimeric) double-stranded nucleic acid formed by hybridisation between target and first probe that is cleaved, there is no need to make use of exonucleases that are chosen on the basis of the constitution of the target DNA, or to use probe nucleic acids that only bind to, for example, DNA or RNA. The improved hybridisation efficiency provided by utilising LNA and PNA can thus be provided by either or both (i) the portion of the target nucleic acid targeted by the targeting region of the first probe or (ii) that targeting region itself comprising PNA or LNA.

Thus is a more generic detection methodology than for just RNA or DNA. The target could be dsDNA using a triplex-forming oligonucleotide (TFO), a protein using an aptamer or an antibody that binds to an appropriate target. In such embodiments, detection can be carried out in the same way as described herein (e.g. for a single-stranded DNA with a probe sequence comprising first and second probe sequences). Thus, with double-stranded DNA as a target nucleic acid, for example, the first probe nucleic acid can be a TFO having a unique sequence forming the triplex, i.e. which targets the double-stranded DNA, and which is conjugated to a sequence of nucleic acid complementary to the second probe nucleic acid. Hybridisation of the second probe nucleic acid and subsequent degradation of the resultant duplex serves to detect the presence of the desired dsDNA without any need for melting.

Analogously, a nucleic acid can be conjugated to an antibody, which may then, for example, be immobilised to a solid support. Detection of a binding event (e.g. to an antigen) may be achieved by a method of the invention in which a nucleic acid which serves as the target nucleic acid can be attached to a further antibody (e.g. in an assay analogous to a sandwich ELISA assay). The presence of the solid-supported antigen can then be determined by detection of the target nucleic acid in accordance with a method of this invention. In this way the methods of the present invention can be used to detect any binding event by conjugating an appropriate nucleic acid as the target nucleic acid to the biomolecular probe in any given study. Binding can then be detected by exposing the biomolecular probe-target nucleic acid conjugate to a probe nucleic acid and practising a method of the invention.

A further advantage of this embodiment of the invention is the ease with which multiplexing is allowed, simply by changing the label in the second probe and the targeting sequence of the first probe. The sequence actually degraded by the exonuclease can remain constant requiring only one set of optimum conditions to be established for the exonuclease can remain constant permitting only set of optimum condition to be established for the exonuclease even in a multiplex array in which a plurality of target nucleic acids are to be detected.

The use of generic exonuclease-cleavable sequences described hereinbefore can also be used in conjunction with the capture methodology described herein. Thus, for example, the portion of the first probe nucleic acid designed to hybridise to the second probe nucleic acid could be derivatised e.g. at the 3'-end with biotin to allow use of the streptavadin-biotin based capture and wash methodologies described herein and with reference to Figure 21. In this way the first probe nucleic acid be serves as a capturable probe as described herein. Use of such methodologies can improve the specificity (and thus reliability) of the methods of this invention (if desired).

An alternative way of improving specificity is depicted in Figure 25. This depicts use of two capture probes which may be attached to a solid surface, e.g. a bead or a plate, or an exemplary alternative derivatised with biotin to allow capture by a streptavadin-coated magnet. Two capture probes are depicted; one or more could be used. Thus where capture methodology is derived to be employed where use as made of generically exonuclease-cleavable sequences the capture probe(s) need not be the same probe as that which hybridises to the labelled probe (as depicted in Figure 24).

In one embodiment of the invention a 5'-phosphate-containing probe nucleic acid specifically hybridised to a target nucleic acid is digested by an exonuclease such as lambda exonuclease, so as to degrade the 5'-phosphate-containing strand. Whilst both strands may be degraded digested, it is sufficient in some embodiments of the invention for digestion of only the probe and release of the Raman/SE(R)RS detectable label.

Lambda exonuclease is a 24kD enzyme encoded by the bacteriophage *Lambda.* This enzyme is implicated in bacteriophage genetic recombination but is also available commercially as a DNA processing enzyme. The crystalline structure of the enzyme has revealed it to be a homotrimer with a toroidal quaternary structure.

Lambda exonuclease digests one strand of double-stranded (dsDNA) starting at a phosphorylated 5' terminus. The dimensions of the central channel of lambda exonuclease are such that it can only accommodate dsDNA at one end of the channel. The enzyme is highly processive, passing the 3' DNA strand through the middle of the tapered channel in the centre of the enzyme. The 5' strand is digested, releasing free 5' mononucleotides. Single-stranded DNA with a phosphorylated 5' terminus has also been shown to be digested but is a poor substrate compared to dsDNA. (see P.G. Mitsis and J.G. Kwagh, Nucleic Acids Research, 27(15), 3057-3063 (1999) and references cited therein).

In some embodiments of the invention, the exonuclease is lambda exonuclease. In other embodiments the exonuclease is not lambda exonuclease. Other exonucleases which are suitable for use in the present invention are exonucleases which are able to progressively digest one or both strands of a nucleic acid duplex in a 5' or 3' direction

whether or not a 5' or 3' terminal nucleic acid is modified, such as by being phosphorylated. It should be appreciated however, that the exonuclease should possess little or no capacity for digesting single-stranded nucleic acid. Other suitable exonucleases include polymerases, such as Taq polymerase, DNA polymerase I, and DNA polyamerase T4.

The probe may be of any convenient length. Its actual length will depend upon how precisely it is desired to determine a given target sequence. Typically, however, the probe will be between about 10 and 50 nucleotides in length, for example 20 to 30 nucleotides in length. The probe is modified so as to allow the change in the environment of the label to be detected upon digestion of the probe by the exonuclease, e.g. lambda exonuclease. Preferably the label is substantially non-detectable when part of the probe, either when the probe is present as a single-stranded nucleic acid and/or when it is hybridised to the target sequence. In this way excess, unhybridized probe, which is much less preferably processed by the exonucleases described, will not be detectable and so the only label detectable will be that present when attached to the probe duplex nucleic acid and has been released on digestion of the duplex nucleic acid. In this way any change in label detected is indicative of the presence of the target sequence in the sample of interest.

In contrast to approaches in the prior art, the probe strand need not necessarily be extended during the methods of this invention. The sensitivity of detection using labels detectable using Raman spectroscopic methods, in particular SE(R)RS, for example, is such that extremely low levels of label may be detected. In certain embodiments of the invention an additional benefit is that once the initial probe has been digested a second probe molecule can bind to the target nucleic acid and be digested to generate more signal from the same fragment of target nucleic acid, on account of the very high turnover rate of certain exonucleases, such as lambda exonuclease. In effect the signal is amplified in these embodiments, as opposed to the target in, for example, PCR. An excess of probe sequence may be used, for example 10 times or more or 100 times or more than the template present, or expected or estimated to be present, in the sample, to ensure the kinetics of hybridisation favour rapid signal generation. This approach also permits multiplexing reactions through the use of differently labelled probes.

However amplification of the target may be effected if desired. Appropriate methods of amplification are known to the skilled person and include isothermal amplification and rolling circle amplification.

In certain embodiments of the invention, fluorescence *per* se as a detection modality may supplement detection by Raman spectroscopy where the label detectable by Raman spectroscopy is fluorescent. In these embodiments the probe will typically comprise a fluorophore and quencher as with molecular beacons or TaqMan probes described hereinbefore. When intact, the quencher will quench any fluorescence from the fluorophore. However, upon degradation of the probe DNA by the exonuclease, after hybridisation to the target nucleic acid, the fluorophore will be released from the quencher allowing fluorescence to be obtained. Where problematic, background fluorescence may be ameliorated by the use of confocal microscopy. This allows ultrasensitive detection approaching the single molecule level. It is also possible to use a FRET arrangement as opposed to quencher-donor and also the use of fluorescence lifetime measurements. Any convenient fluorophore may be used. In one embodiment fluorescein-dT (emission at 516 nm) may be used. Fluorescein-dT is a modified base wherein fluorescein (FAM) is attached to position 5 of the thymine ring by a 6-carbon spacer arm. Any convenient quencher may be used, for example Dabcyl (which quenches at 380-530 nm).

In another embodiment, the detection modality is based upon plasmonics, which relies upon nanoparticle plasmon resonance as an indicator of hybridisation, and so the change in aggregation state of nanoparticles in solution after the action of the exonuclease. According to this embodiment individual nanoparticles are functionalised with the probe such that a terminal phosphate (e.g. 5'-phosphate where λ exonuclease is used as the exonuclease) or nucleotide base is distal to the surface of the nanoparticle. When functionalised the probe will have a particular resonance frequency. When these probes hybridise to the target region the terminal phosphate/base acts as a recognition site for the exonuclease to allow digestion of the probe on the surface of the nanoparticle. Whilst nanoparticles coated with probe sequence are isolated and unaggregated in solution, upon hybridisation to the target sequence and subsequent degradation of the probe sequence the nanoparticles will no longer be held apart and will aggregate resulting in a detectable change.

In one embodiment, a surface may be provided to which a probe nucleic acid is attached. The surface is one which is suitable for use in Raman (SE(R)RS detection and may be a roughened metal surface, such as a silver surface. The surface may be for example, the surface of a nanoparticle, microtitre plate, microarray surface or the like. The Raman (e.g. SE(R)RS) detectable label may be brought down onto the surface upon digestion of the nucleic acid duplex.

Also, if the surface is the surface of a nanoparticle, the probe would prevent the particles from aggregating, but upon probe digestion, the particles may aggregate, thereby enhancing any detectable Raman (e.g. SE(R)RS) signal.

If sufficiently small numbers of probe are attached to the nanoparticle surface (e.g. 10 - 20 % surface coverage) then it may be assumed that the aggregation state of the nanoparticles will change as the probes are degraded and give rise to a change in plasmon frequency of the nanoparticles. This may be measured using, for example, dark field microscopy (see for example Homogeneous detection of unamplified genomic DNA sequences based on colimetric scatter of gold nanoaprticle probes (J. J. Storhoff et al., Nature Biotech. 2004, 22(7), 883-887)).

In accordance with this embodiment a SE(R)RS active dye (a SE(R)RS label) may be directly attached to the nanoparticle, but when the probe is still intact, the label is invisible as there is no aggregation. The digestion of the probe/target duplex allows aggregation of the particles to occur and also switches on the SERRS effect.

Gold or silver colloids may be used as the nanoparticles. These colloids can be considered as sols i.e. solid particles dispersed in water. The size of these particles is on the nano-scale, hence the use of the term nanoparticles typically 2 - 100 nm in particular 10 - 80 nm, such as 15 - 40 nm). The colloidal medium is well suited to DNA hybridisation reactions as oligonucleotides can be immobilised on the surface of the gold or silver nanoparticles.

In preferred embodiments, as discussed hereinbefore, the detection modality is based upon SE(R)RS. Details about SERS and SERRS are set forth in, for example, WO97/05280, WO99/60157 and WO2005/019812 and references cited therein.

As is known in the art, a Raman spectrum arises because light incident on an analyte is scattered due to nuclear motion and excitation of electrons in the analyte. Where the analyte whose spectrum is being recorded is closely associated with an appropriate surface, such as a roughened metal surface, this leads to a large increase in detection sensitivity, the effect being more marked the closer the analyte sits to the "active" surface (the optimum position is in the first molecular layer around the surface, i.e. within about 2 nm of the surface). This is termed SERS.

A further increase in sensitivity can be obtained by operating at the resonance frequency of the analyte (in this case usually a dye attached to the target of interest). Use of a coherent light source, tuned to the absorbance maximum of the dye, gives rise to a $10^3$-$10^5$-fold increase in sensitivity (the laser excitation may also be set to the maximum of the surface plasmon resonance, which may or may not coincide with the dye maxima). The surface enhancement effect and the resonance effect may be combined to give SERRS and a range of excitation frequencies will still give a combined enhancement effect.

Thus the technique of SERRS provides a vibrational fingerprint of the analyte when two conditions are met. These are (i) the adsorption onto a suitable metal surface and (ii) the presence of a visible chromophore. The use of a metal additionally means that fluorescence is efficiently quenched. This means that a large range of coloured molecules, including standard fluorophores, give excellent SE(R)RS signals.

SERRS is generally preferred, and so all reference herein to SE(R)RS may be read onto SERRS unless the context specifically indicates to the contrary. However it will be understood the invention can also be practised with SERS, and with Raman spectroscopy in general. SERS is advantageous, for example when minimising background fluorescence by using an excitation frequency in the infra-red region.

An example of how to generate a label which is invisible to SE(R)RS, but then becomes visible after the action of an enzyme, is described for example, in Moore et al. (2004 Nature Biotech., 22, p1133-1138). In this example a lipase is used to hydrolyse an ester linkage which releases a dye that becomes SE(R)RS active. In furtherance of this, as applied to the present invention, a hydroxyquinoline azo dye may be attached to the 3'-phosphate of the probe nucleic through the phenolic group of the dye. This masks the dye from adhering to a metal surface and makes the dye "invisible" to SERRS. In this approach once the probe has hybridized to its specific sequence, the exonuclease will digest the oligonucleotide and release the hydroxyquinoline dye which can then be detected by SERRS, by virtue of the dye being able to adhere to a SE(R)RS active surface.

Due to the extreme sensitivity of SE(R)RS, amplification of template may not be required in many instances, to make this process work. However amplification of the target may be effected if desired. Appropriate methods of amplification are known to the skilled person and include isothermal amplification and rolling circle amplification.

In accordance with a further embodiment, a probe may be used that has a 5'-modified SE(R)RS active dye attached to it. The dye may be attached in such a way that the probe will not stick down on the metal surface and give SERRS. After formation of the probe/target duplex and addition of an exonuclease, such as Taq, degradation occurs and the label is released. This probe is degraded in a similar manner to a Taqman probe; however rather than removal of fluorescence quenching and an increase in fluorescence upon the action of the enzyme, in this embodiment of the present invention the ability of the dye to stick to the metal surface increases and as such gives an increase in the SERRS signal. As described hereinbefore this embodiment may be practised in conjunction with a use of a capturable moiety such as biotin to allow the removal of unbound or excess probe. With either of these, or other, embodiments of the invention, such probes may be deployed in combination with amplification of nucleic acid present in the sample of interest, e.g. by PCR.

Conveniently, where amplification techniques are used in conjunction with Raman-detectable probes, the probes used may be designed to hybridise to a different portion of target nucleic acid to that hybridised by, for example, primers used in PCR. In this way, action of the exonuclease, e.g. Taq, upon the primers continues into the probe, if hybridised, releasing the Raman-detectable label.

Another method of practising the invention involves the use of a SE(R)RS active intercalator or, preferably, a minor groove binder (MGB) which if attached to the probe sequence would increase the specificity of the hybridisation. When the probe sequence is hybridised to the target, the intercalator or MGB is designed such that no SE(R)RS is obtained; however upon digestion by the exonuclease it is released into solution and is able to adsorb onto a metal surface and give SE(R)RS.

## Oligonucleotides used

The oligonucleotides were purchased from atdbio (England) and MWG (Germany) and were HPLC purified.

| Name | Sequence | Source | Conc./ μM | Modifications |
|---|---|---|---|---|
| RW01A | 5'-TTTTCCCAGTCACGACGT-3' | atdbio | 41.17 | 5' Phosphate<br>3' Thiol |
| RW01B | 5'-TTTTCCCAGTCACGACGT-3' | atdbio | 63.17 | 5' Phosphate<br>3' Thiol |
| RW02A | 5'-TTTTCCCAGTCACGACGT-3' | atdbio | 85.73 | 5' Phosphate<br>3' Amino linker |
| RWFAM | 5'-TTTTCCCAG*TCACGACGT-3' | atdbio | 18.31 | 5' Phosphate<br>3' Dabcyl<br>***T** Fluorescein dT |
| RWCOMP1 | 5'-ACGTCGTGACTGGGAAAA-3' | atdbio | 31.47 | - |
| RWCOMP2 | 5'-<br>ACGTCGTGACTGGGAAAACC<br>CTGGCGTTACCCAACTTA-3' | MWG | 29.60 | - |
| RWCOMP3 | 5'-TCACTGGCCGTCGTTTTACA<br>ACGTCGTGACTGGGAAAA | MWG | 32.30 | - |
| RWCOMP4 | 5'-TCACTGGCCGTCGTTTTA<br>CAACGTCGTGACTGGGAA<br>AACCCTGGCGTTACCCAA-3' | MWG | 14.40 | - |

## UV melt of Fluorescent Probe and Complementary Strand

A UV-melting curve of the fluorescein labelled oligonucleotide (RWFAM) and the complementary sequence (RWCOMP1) was obtained using a Cary 300 Bio UV-Vis spectrophotometer. The melting curve was used to find the melting temperature of the two strands.

54.6 μl of RWFAM and 31.8 μl of RWCOMP1 were added to 1913.6 μl of 0.3 M PBS in a quartz glass cuvette. This gave an overall concentration for each oligo of 0.5 μM in an overall volume of 2 ml.

The UV melt was carried out in 4 ramps with a 1 minute hold after each stage:

- Ramp 1: 25 °C → 90 °C
- Ramp 2: 90 °C → 25 °C
- Ramp 3: 25 °C → 90 °C
- Ramp 4: 90 °C → 25 °C

The 1$^{st}$ derivative curves of each ramp were used to find the mean melting temperature of RWFAM and RWCOMP1.

## Fluorescence Probe Experiments Introduction

## Outline

These experiments involved using an oligonucleotide probe modified with a fluorophore and quencher. The probe was designed to hybridize to a series of complementary target oligonucleotides and in doing so, become susceptible to the action of lambda exonuclease. Any degradation of the probe was detected by an increase in fluorescence.

**Oligonucleotide Dilutions**

The following table lists dilutions of the stock solutions of oligonucleotides that were used in these experiments.

| Name | Dilution Factor | Diluted Concentration |
|---|---|---|
| RWFAM | 0 (Stock) | 18.31 μM |
| | 10 | 1.8 μM |
| | 100 | 0.18 μM |
| | 1000 | 18 nM |
| | 10000 | 1.8 nM |
| RWCOMP1 | 0 (Stock) | 31.47 μM |
| | 10 | 3.1 μM |
| | 100 | 0.31 μM |
| | 1000 | 31 nM |
| | 10000 | 3.1 nM |
| RWCOMP2 | 0 (Stock) | 29.6 μM |
| | 10 | 2.9 μM |
| RWCOMP3 | 0 (Stock) | 32.3 μM |
| | 10 | 3.2 μM |
| RWCOMP4 | 0 (Stock) | 14.4 μM |
| | 10 | 1.4 μM |

**Enzyme Specifications**

- Lambda Exonuclease purchased from Epicentre (Cambio)
- Enzyme Storage Buffer: 50% glycerol solution containing 50 mM Tris-HCl (pH 7.5), 100 mM NaCl, 1.0 mM DTT, 0.1 mM EDTA and 0.1% Triton®X-100.
- On receiving enzyme it was aliquoted into 25x 10 μl portions and stored in a freezer. Each 10 μl portion contained 100 units of the enzyme.

**Lambda Exonuclease Reaction Buffer**

- 10X Reaction Buffer: 670 mM Glycine-KOH (pH 9.4), 25 mM $MgCl_2$ and 0.1% Triton® X-100
- Reaction buffer was split into 150 μl portions.

**Instrumentation**

- All reactions carried out on a Stratagene MX4000 QPCR.

**Sample Preparation**

- All samples were prepared in a clean environment within a sterile glove box.
- Samples were prepared in sterilised 200 μl tube strips.
- Samples were prepared to a total volume of 150 μl.
- Due to the micro-scale volume of some of the reaction components used, the samples were briefly centrifuged between each stage. This ensured that all the reagents were mixed and were not adhering to the sides of the tubes out with the main body of the sample.

**Experimental Procedure**

Experiments with the fluorescent probe were conducted using a Stratagene MX4000 QPCR workstation and involved two stages:
1. The reaction mixtures without the enzyme were heated to 90 °C and held at this temperature for 10 minutes to ensure separation of the DNA strands. The probe and target strands were allowed to hybridize by cooling the samples to 20 °C and holding at this temperature for 5 minutes. The temperature was brought back up to 37 °C in preparation for the second stage.
2. The enzyme (or water as a control) was added to reaction mixtures. The samples were heated to 37 °C and the fluorescence measured. Three slightly different programmes were used:

| Programme | Number of Cycles | Cycle Duration / minutes | Total Duration / minutes |
|---|---|---|---|
| A | 40 | 2 | 80 |
| B | 40 | 1 | 40 |
| C | 60 | 1 | 60 |

Triplicate fluorescence measurements were made at the end of each cycle.

**Fluorescence Experiments**

The data presented below was normalised by subtracting the initial fluorescence value from each data point. Where measurements were taken in duplicate, only the mean value is shown.

**Experiment 1: Initial Test**

This experiment was conducted to confirm the feasibility of the technique. It is described here in full as an example of the procedure also used in Experiments 2 and 3.

The following samples were prepared in duplicate in separate tubes of an 8-tube strip:

| **Test Samples:** 0.1 μM dsDNA + 10U λExo enzyme | | | **Control Samples:** 0.1 μM dsDNA, no λExo enzyme | | |
|---|---|---|---|---|---|
| Component | Conc./ μM | Volume / μl | Component | Conc./ μM | Volume / μl |
| RWFAM | 1.8 | 8.2 | RWFAM | 1.8 | 8.2 |
| RWCOMP1 | 3.1 | 4.8 | RWCOMP1 | 3.1 | 4.8 |
| 10X Reaction Buffer | - | 15 | 10X Reaction Buffer | - | 15 |
| Sterile Water | - | 121 | Sterile Water | - | 122 |

Two test samples were prepared to give a 0.1 μM concentration of dsDNA once hybridised. RWFAM (8.2 μl, 1.8 μM) and RWCOMP1 (4.8 μl, 3.1 μM) were added to 10X reaction buffer (15 μl) in 121 μl of sterile water.

Duplicate control samples were prepared using the same volumes and concentrations of oligonucleotides and reaction buffer but with 122 μl of sterile water.

The samples were placed in the QPCR instrument for the hybridisation stage of the procedure. Following hybridisation, the samples were removed and 1 μl (10 Units) of lambda exonuclease added to the two test samples. The samples were returned to the QPCR instrument and the fluorescence measured using programme A.

The results are shown in Figure 4 which shows an increase in fluorescence occurred only in the samples to which lambda exonuclease was added. This indicates that the enzyme was able to degrade the probe and release the

fluorophore from the quencher. A constant level of fluorescence was obtained after 50-60 minutes. The control samples remained at the baseline.

**Experiment 2**

This investigated the effect of doubling the concentration of lambda exonuclease.
The following test samples and controls were prepared in duplicate:

| **A)** 0.1 µM dsDNA, no λExo | | | **B)** 0.1 µM dsDNA + 10U λExo | | |
|---|---|---|---|---|---|
| Component | Conc. / µM | Volume / µl | Component | Conc. / µM | Volume / µl |
| RWFAM | 1.8 | 8.2 | RWFAM | 1.8 | 8.2 |
| RWCOMP1 | 3.1 | 4.8 | RWCOMP1 | 3.1 | 4.8 |
| 10X Reaction Buffer | - | 15 | 10X Reaction Buffer | - | 15 |
| Sterile Water | - | 122 | Sterile Water | - | 121 |
| **C)** 0.1 µM dsDNA + 20U λExo | | | **D)** 100% Water | | |
| Component | Conc. / µM | Volume / µl | Component | Conc. / µM | Volume / µl |
| RWFAM | 1.8 | 8.2 | RWFAM | - | - |
| RWCOMP1 | 3.1 | 4.8 | RWCOMP1 | - | - |
| 10X Reaction Buffer | - | 15 | 10X Reaction Buffer | - | - |
| Sterile Water | - | 120 | Sterile Water | - | 150 |

The samples were prepared and hybridised as described in Experiment 1. Following hybridisation, the samples were removed from the QPCR instrument.

1 µl of lambda exonuclease was added to samples A & B.
2 µl of lambda exonuclease was added to sample C.

Samples of D were used as a control.
The samples were returned to the QPCR instrument and the fluorescence measured using programme C.
The graph in Figure 5 again shows that no change in fluorescence occurs in the control samples. The data shows that when the number of units of enzyme used is doubled, a more rapid increase in fluorescence occurs and that the maximum level of fluorescence is reached in a shorter period of time. The data shows that the time taken to degrade the same concentration of fluorescent probe is approximately proportional to the concentration of enzyme present. For 0.1 µM concentration of the fluorescent probe, using twice the number of units of lambda exonuclease appears to half the time taken to reach the maximum level of fluorescence.

**Experiment 3**

This investigated the effect of recessed 5' ends and 3' tails on the ability of lambda exonuclease to degrade the probe strand and used three different target strands:

- RWCOMP2 - provided a 20 base 5' recess
- RWCOMP3 - provided a blunt 5' end a 20 base 3' tail.
- RWCOMP4 - provided both a 20 base 5' recess and a 20 base 3' tail.

Duplicates of the following samples were prepared:

| **A)** 20 base 5' Recess | | | **B)** Blunt 5' end and 20 base tail | | |
|---|---|---|---|---|---|
| Component | Conc. / µM | Volume / µl | Component | Conc. / µM | Volume / µl |
| RWFAM | 1.8 | 8.2 | RWFAM | 1.8 | 8.2 |

(continued)

| **A)** 20 base 5' Recess | | | **B)** Blunt 5' end and 20 base tail | | |
|---|---|---|---|---|---|
| Component | Conc. / µM | Volume / µl | Component | Conc. / µM | Volume / µl |
| RWCOMP2 | 3.0 | 5.0 | RWCOMP3 | 3.2 | 4.7 |
| 10X Reaction Buffer | - | 15 | 10X Reaction Buffer | - | 15 |
| Sterile Water | - | 120.8 | Sterile Water | - | 121.1 |
| **C)** 20 base 5' Recess and 20 base tail | | | **D)** 0.1 µM dsDNA + 10U λExo | | |
| Component | Conc. / µM | Volume / µl | Component | Conc. / µM | Volume / µl |
| RWFAM | 1.8 | 8.2 | RWFAM | 1.8 | 8.2 |
| RWCOMP4 | 1.4 | 10.7 | RWCOMP1 | 3.1 | 4.8 |
| 10X Reaction Buffer | - | 15 | 10X Reaction Buffer | - | 15 |
| Sterile Water | - | 115.1 | Sterile Water | - | 121 |
| **E)** Control: Water | | | | | |
| Component | Conc. / µM | Volume / µl | | | |
| RWFAM | - | - | | | |
| RWCOMP_ | - | - | | | |
| 10X Reaction Buffer | - | - | | | |
| Sterile Water | - | 150 | | | |

The samples were prepared and hybridised as carried out previously. Following hybridisation, the samples were removed from the QPCR instrument and 1 µl (10 Units) of lambda exonuclease added to samples A, B, C and D.

The samples were returned to the QPCR instrument and the fluorescence measured using programme C.

The aim of this experiment was to determine whether lambda exonuclease would be able to degrade the fluorescent probe when hybridised to target DNA of a longer chain length. This would cause the 5' end of the probe to be recessed in comparison to the target strand and would also give a long tail section. This was intended to reflect a real-life DNA sample in which the complementary target sequence would be part of a much longer nucleic acid chain.

The experimental data (shown in Figure 6) shows that form of the duplex between the probe and target strand does not really affect the overall activity of the enzyme as the overall fluorescence increase obtained in each case was similar.

**Chemicals and reagents:**

Chemicals were purchased from either Sigma or Aldrich and were all analytical grade.

Lambda exonuclease enzyme (2500 units, 10 units µl, *Ref. LE032K*) and lambda exonuclease reaction buffer (670 mM Glycine-KOH, 25 mM $MgCl_2$, 0.1 % Triton X-100, x10 stock, *Ref. LE-buffer)* were obtained from Cambio Ltd., U.K. Lambda Exonuclease was supplied in a 50% glycerol solution containing 50 mM Tris-HCl (pH 7.5), 100 mM NaCl, 1.0 mM dithiothreitol, 0.1 mM EDTA and 0.1% Triton® X-100.

Streptavidin-coated magnetic beads were purchased from New England Biolabs. Buffers were filtered through a 0.2 µm pore size syringe filter (Whatman) prior to use.

**Oligonucleotide sequences:**

Oligonucleotides were purchased from ATDbio and MGW.

**A2020** / PTBPROBE (18 mer)

5'- TTT TCC CAG TCA CGA CGT

| | | |
|---|---|---|
| *Modifications:* | *5'* | *phosphate* |
| | *Mid (10)* | *dT TAMRA* |
| | *3'* | *Biotin* |

**A2020** / PFBPROBE (18 mer)
5'- TTT TCC CAG TCA CGA CGT

| | | |
|---|---|---|
| *Modifications:* | *5'* | *phosphate* |
| | *Mid (10)* | *dT FAM* |
| | *3'* | *Biotin* |

**A0977/** Complementary sequence (18 mer) -for both sequences above
5'- ACG TCG TGA CTG GGA AAA
**CT sequences**
**A2057** / CTPROBE (22 mer)
5'- GCT AAA CTT GCT TGC CAC TCA T

| | | |
|---|---|---|
| *Modifications:* | *5'* | *phosphate* |
| | *Mid (12)* | *dT FAM* |

**A2058** / CTCOMP (22 mer)
5' - ATG AGT GGC AAG CAA GTT TAG C
**First Strand CT** (100 mer amplicon)

GGA ATT TCC ACT TGA TAT TAC CGC AGG AAC AGA AGC TGC GAC AGG GAC TAA GGA TGC CTC TAT TGA CTA CC**A TGA GTG GCA AGC AAG TTT AGC** CCT TTC T

*Binding site for the FAM probe is shown in bold.*
5' 7 base overhang
3' 71 bases

**Instrumentation**

SERRS spectra were collected using a Renishaw *In-Via* Raman microscope system incorporating an argon ion laser operating at 514.5 nm and power of 30 mW. A silicon standard was used to calibrate the instrument by measuring the intensity of the 520 $cm^{-1}$ peak.

Fluorescence measurements were carried out on a Stratagene MX400 Q-PCR which uses a Quartz-Halogen lamp as the light source. Bandpass filters were generally set at EX. 350 nm, EMM. 516. All other channels were blocked by using light blocking filters. A Cary Eclipse spectrophotometer was also used for general fluorescence measurements.

**<u>Assay using lambda exonuclease</u>**

The following assay was based on the use of a commercially available 18-mer labelled with a 5'-phosphate, an internal TAMRA dT modification and a 3' terminal biotin as the degradable probe (in accordance with schematic shown in Figure 3).

PTBPROBE = 5' Phosphate-TTT TCC CAG T(X) CA CGA CGT-Biotin 3'

where X= TAMRA modification of adjacent T

Briefly, the reaction mixtures (9-10 μl, 1 picomole of PTBPROBE in the presence /absence of 1 eq. of complementary ssDNA) were heated to 90 ºC and cooled to 20 ºC to promote the hybridisation of the complementary sequences. The lambda exonuclease enzyme was then added and the reaction was incubated at 37 ºC for 30 mins to initiate digestion. This enzyme is a highly processive 5' to 3' exodeoxyribonuclease that releases 5'-mononucleotides and the non-hydrolyzed complementary single-stranded DNA (ssDNA) strand as products. A TAMRA-labelled mononucleotide should be released upon digestion of the PTBPROBE described above.

Following digestion, the reaction mixtures were heated to 75 ºC for 15 minutes to fully inactivate the enzyme and essentially quench the reaction before cooling the samples back to room temperature. Streptavidin-coated magnetic beads in washing and binding buffer (0.5 M NaCl, 20 mM Tris-HCl, 1 mM EDTA, pH 7) were then used to remove any undigested PTBPROBE via the strong interaction with the 3' biotin modification on the labelled oligonucleotide. This step was introduced to remove undigested, dye labelled probe which could lead to background signals. A magnet was used to pull the magnetic beads to the side of the eppendorf and the supernatant was then transferred to a clean microtitre well plate. An aggregating agent (20 μl) was added followed by citrate-reduced Ag colloid (100 μl). SERRS spectra were recorded immediately after the addition silver colloid on a InVia Raman Microscope using a 514 nm excitation laser line.

Lambda exonuclease is a highly processive 5' to 3' exodeoxyribonuclease that releases 5'-mononucleotides and the non-hydrolyzed complementary single-stranded DNA (ssDNA) strand as products. Dye-labelled mononucleotides should be released upon digestion.

*SERRS detection strategy:*

The reaction mixture contains a complex combination of enzyme, full length oligonucleotides, truncated digestion products and a large number of compounds.

The approximate concentrations of the different species in the reaction mixture (20 μl, including the biotin wash step) *before* SERRS analysis are listed below:

**0.1 μM oligonucleotide labelled probe (initially, plus digestion products)**
**0.1 μM complementary oligonucleotide target**
33.5 mM Glycine.KOH,
1.25 mM $MgCl_2$
0.01% TX-100 (surfactant)
0.255 M NaCl,
12.5 mM Tris-HCl
0.505 mM EDTA
2.5 % Glycerol
0.05 mM DTT
10 units of lambda exonuclease enzyme

Glycine, a simple amino acid, is present at the high concentrations and it is known to interact with the surface of citrate-reduced silver colloid (Surface-enhanced Raman scattering of biological molecules on metal colloid II: effects of aggregation of gold colloid and comparison of effects of pH of Glycine solutions between gold and silver colloids: X. Dou, Y. M. Jung, Z. Cao and Y. Ozaki, Appl. Spectrosc., 1999, 53, 1140). The extent of its interaction with the silver surface is affected by pH, and this may be correlated to the pKa values of the different ionisable species:

**pKa values of stabilising species in silver and gold colloids:**

Citrate: 3.1, 4.7, 6.4
EDTA: 2.0, 2.7, 6.1, 10.2
Glycine: $pK_1$ = 2.34 (COOH), $pK_2$ = 9.6 ($NH_3^+$) Isoelectric point is 5.97

The digestion reaction was carried out at pH 9.4 (buffered) but addition of citrate-reduced silver colloid (pH 6-7) might have lowered the overall pH to some degree. In any case, this suggests that the citrate groups on the silver colloid would be fully deprotonated under these conditions. Furthermore, a large proportion of the glycine should be in the zwittzerionic ($NH_3^+$, $COO^-$) at the time of analysis.

When exonuclease reaction buffer was added to silver nanoparticles (in the same ratio as that used in the actual assay but different volume) a decrease in -ve Zeta-potential was observed (Table 1). This suggests a change in the surface charge of the particles. However, the particles showed no apparent change in aggregation at this stage.

**Table. 1.** pH and Zeta potential values for silver colloids in $H_2O$ and exonuclease reaction buffer (Gold not used in this study but included for comparison).

| Colloid Sample | pH | Zeta Potential / mV | |
|---|---|---|---|
| | | Batch Produced | In reaction buffer |
| Silver / citrate reduced | 6 | - 51.5 ± 2.5 | - 31.4 ± 0.9 |
| Silver / edta reduced | 10-11 | - 47.2 ± 1.4 | -38 ± 2.7 |
| Gold / citrate reduced | 5-6 | - 41.8 ± 2.4 | - |

This serves to highlight the importance of pH and its effect on some of the different species in the reaction mixture. pH will likely dictate the affinity of different chemicals to the metal surface and this in turn will affect any subsequent SERRS studies.

The use of $MgSO_4$ to aggregate the SERRS particles proved successful for our purposes when using the TAMRA-labelled oligonucleotide probe (Figure 7 and 8). It is possible to obtain the SERRS signal of the released TAMRA label although some background signals from the control samples were also observed. However, in a study using a series of triplicate reactions is was possible to establish a large degree of discrimination between the control samples and the positive test reaction containing the target sequence.

Brief experimental:

PTBPROBE (5'-phosphate, internal dT(TAMRA), 3' Biotin) was mixed with its complementary sequence in lambda exonuclease reaction buffer (67 mM Glycine-KOH, 2.5 $MgCl_2$, 0.01% TX-100), heated to 90 ºC and then cooled to 20 ºC over a period of ~ 15 mins (0.1 μM, 1:1 ratio, 10 μl reaction scale, 1 picomole dsDNA). Lambda exonuclease (1 μl, 10 units) was added and digestion was carried out at 37 ºC for 30 minutes followed by an inactivation step (75 ºC for 15 minutes). The reaction mixture was transferred to a 96-well microtitre plate and incubated with streptavidin-coated magnetic beads (10 μl, 4 mg / ml) for 30 minutes at room temperature on a rotary shaker. The microtitre plate was then placed on a magnetic stand to separate the magnetic beads towards the side of the well and the supernatant (20 μl) was then transferred into a clean well. An aggregating agent (20 μl, 1 M $MgSO_4$) was added followed by 100 μl of citrate-reduced silver colloid. SERRS spectra were acquired immediately afterwards (Renishaw in Via Raman, 514.5 nm, x10 objective, 100% power, 10 sec, 3 accu.). (See Figure 9)

__A Fret-Based Assay__

Schematic representations of assays of this type are shown in Figure 10.

A reaction was carried out on a 10 μl scale using different ratios of FAM-labelled probe (5' phosphate - TTT TCC CAG (AT-FAM) CA CGA CGT - Biotin)to complementary target sequence (1:1; 10:1, 100:1). The concentration of FAM-labelled probe and H33258 dye (M. Teng, N. Usman, C.A. Frederick and H.J. Wang, Nucl. Acids Res., 1988, 16 No. 6; D.E. Wemer, Biopolymers, 1999, 52, 197-211; and F.M. Ho & E.A.H Hall, Biosensors and Bioelectronics, 2004, 20, 5, 1001-1010) was 1 μM. Lambda exonuclease reaction buffer (1 μl, 10 units) was added and the reaction mixture was heated to 37 ºC for 30 mins followed by an enzyme inactivation step at 75 ºC for 15 mins. A solution containing complementary target sequence (100 μl, 0.1 μM) was added to each reaction and melting/annealing curves were carried out (temperature range 15-75 ºC, monitoring changes in fluorescence @ $Ex_{350\ nm}$, $Emm_{520\ nm}$ every 1 ºC, x3 annealing curves and x3 melting curves). Control samples (no enzyme added) showed $T_m$ ~ 58 ºC which is consistent with UV-melting data. None of the digested samples showed discernable melting curves which suggest that the FAM-labelled probe was digested in every case.

These results are shown in Figures 12 and 13.

__Further Examples__

***1. MATERIALS***

DNA sequences were purchased from ATDbio. The DNA sequences used are listed in table 2.

***Table 2:*** DNA probes used for exonuclease assay

| Name | Nucleotide sequence | 5' mod. | Mid mod. | 3' mod. |
|---|---|---|---|---|
| **A1220**/FAM probe | 5´ TTT-TCC-CAG-TCA-CGA-CGT 3´ | Phosphate | FAM base 10 | biotin |
| **A1219**/TAMRA probe | 5' TTT-TCC-CAG-TCA-CGA-CGT 3' | Phosphate | TAMRA base 10 | biotin |
| **A1710**/no FAM probe | 5´ TTT-TCC-CAG-TCA-CGA-CGT 3´ | Phosphate | - | - |
| **A0977**/perfect complement | 5´ ACG-TCG-TCA-CTG-CGA-AAA 3´ | - | - | - |
| **LE1/**3'overhang | 5´ ACG-TCG-TCA-CTG-CGA-AAA-CCC-TGG-CGT-TAC-CCA-ACT-TA 3´ | - | - | - |
| **LE2**/5'overhang | 5´ TCA-CTG-GCC-GTC-GTT-TTA-CAA-CGT-CGT-CAC-TGC-CGA-AA 3´ | - | - | - |
| **LE3**/overhang both sides | 5´ TCA-CTG-GCC-GTC-GTT-TTA-CAA-CGT-CGT-CAC-TGC-CGA-AAC-CCT-GGC-GTT-ACC-CAA-CTT-A 3´ | - | - | - |

Hoechst dye H33258 was purchased from Sigma-Aldrich.
Quant-iT™ PicoGreen ® reagent was purchased from Invitrogen.
Recombinant lambda exonuclease and x10 exonuclease reaction buffer were purchased from Cambio. When diluted to x1, the composition of this buffer is 67 mM Glycine-KOH, 2.5 mM $MgCl_2$, 0.1% Triton X-100; pH 9.4 at 25°C
Miscellaneous chemicals were purchased from Sigma-Aldrich.
Phosphate buffer used for the experiments was 6 mM phosphate with 0.3 M NaCl.

### *2. INSTRUMENTATION*

Absorption studies and UV-melting were carried out on a *CARY 300 Bio* UV-visible spectrophotometer. Emission studies were performed with a *Varian CaryEclipse* fluorescence spectrophotometer. In both cases optically-clear plastic cuvettes of 1 cm path length and 3 ml volume were used. Enzymatic assays were performed using a *Stratagene Mx4000* PCR instrument.

### *6. EXONUCLEASE ASSAY*

Samples were prepared in x1 lambda exonuclease reaction buffer with a final volume of 150 μl. The concentration of sequence, complement and/or Hoechst dye was 1μM in every case. In the samples with enzyme, 10 units of λexo (1μl of 10 units/μl concentration) were added to the reaction mixture. The excitation wavelength was set at 350 nm (10 nm band pass) and the emission measurements were taken at 519 nm for the FRET system and 440 nm for Hoechst dye fluorescence (10 nm band pass). Samples were placed in a cool plate during preparation to avoid early digestion.

Samples were held at 37ºC for 30 minutes (digestion stage) measuring fluorescence every minute. Then the enzyme was denaturalised (inactivation stage) at 75ºC for 15 minutes. Three melting stages were performed afterwards: the annealing curve from 75 to 20ºC, then heat again to 75ºC and a last cooling stage to 25ºC. Heating or cooling rate

was 1ºC/min measuring fluorescence every minute.

The same assay was used for the PicoGreen-TAMRA system using samples of 0.1 μM concentration of TAMRA probe and complementary sequence and 1/10000 dilution of PicoGreen. Samples were 150 μl volume in λexo reaction buffer. Excitation wavelength 492 nm (10 nm band pass), emission measured at 515 nm (10 nm band pass).

## V. RESULTS AND DISCUSSION

### *4. EXONUCLEASE ASSAY*

4.1. DIGESTION OF FAM PROBE

The digestion with lambda exonuclease was performed as described in the experimental section. Two controls were used:

- Control single-stranded DNA: sample containing 1 μM FAM probe and 1 μM H33258 in exonuclease reaction buffer in presence of the enzyme. We will assume the fluorescence of this control to equal 100% of DNA digested.
- Control double-stranded DNA: sample containing 1 μM FAM probe, complement and Hoechst, but not containing exonuclease. This will be assumed to equal 0% digestion.

Figure 14 shows the fluorescence of both controls and of the sample digested within the 30 min digestion (showing data from one of three replicates). For the control samples there is no appreciable change in fluorescence intensity. However, the emission of hybridised FAM probe displays an excellent decay, starting from the level of 100% double-stranded DNA and reaching the values of single-stranded. We can see the progressive decrease in the fluorescence intensity with time, showing the ability of this FRET system to monitor enzymatic digestion in real time.

The annealing curves performed on the samples afterwards (figure 15) provide more evidence on the digestion. Sample without enzyme rehybridises when cooled, showing a Tm of around 5ºC, the same as observed in previous studies for FAM probe. Sample digested with lambda exonuclease shows no ability to rehybridise, meaning that the digestion has actually degraded FAM probe completely.

4.2. DIGESTION OF FAM PROBE USING DIFFERENT COMPLEMENTS

The capacity of lambda exonuclease to digest DNA when it is hybridised with longer sequences than the perfect complement was investigated. For this, the same enzymatic reaction was carried out for the following samples:

- Control single-stranded DNA (FAM probe)
- Perfect complement: FAM probe, perfect complement and Hoechst dye, 1 μM each in λexo reaction buffer
- 3' overhang: same but using a 12 base-pair longer in the 3' end complementary sequence
- 5' overhang: same but with the overhang in the 5' end
- Overhang both sides: 12 base-pair overhang in both 3' and 5' ends

For each of the above, one set of reactions (three replicates per type of sample) was used as the control double-stranded DNA (without the enzyme), and another one was assayed with lambda exonuclease as described previously.

The appearance of the fluorescence vs. time plots is very similar to the one for the perfect complement (graphs not shown), in every case showing a marked decrease in the intensity (see figure 16). Melting curves performed afterwards showed all samples had been digested.

We can conclude after these results that lambda exonuclease is able to digest DNA even when the complementary sequence is longer than the probe.

**Use of dual-labelled probe to detect a human pathogen in a biological sample by SERRS.**

The single tube detection of a human pathogen from a biological sample by SERRS was carried out for *Chlamydia trachomatis.* Using a technique analogous to *Taq*-Man a region of the *ompA* gene (GenBank seq. AY535172) was amplified by PCR with the concomitant digestion of a dual-labelled probe. The data below shows that it is possible to distinguish between *C. trachomatis* positive and C. *trachomatis* negative samples based upon the intensity of a SERRS signal. In this example the SE(R)RS active rhodamine dye R6G was used; however any SE(R)RS (or other Raman) active label could be used. The probe/primer set that was used in this assay was based upon a clinical diagnostic PCR used for the identification of chlamydia infections. The samples were provided by the QCMD program (Quality Control for Molecular Diagnostics) in the form of lyophilised urine spiked with various concentrations of C. *trachomatis* genomic

DNA. The C. *trachomatis* DNA was derived from 3-day cultures grown on McCoy cells which were harvested and heat-inactivated.

**Materials and Methods**

Reconstitution of samples

Samples were received as sterile rubber sealed vials containing lyophilised urine and *Chlamydia trachomatis* genomic DNA. These were injected with 1.2 mL sterile milliQ water and rocked at 37 °C for 1 hour to reconstitute the samples.

Preparation of template

The entire sample was removed from the vial to a sterile 1.5 mL tube and yeast tRNA (Sigma) added to a final concentration of 100 $\mu g.mL^{-1}$. Two volumes of ice-cold ethanol and 1/10 volume of 3 M sodium acetate were added to the sample. The sample was mixed thoroughly and incubated at -20 °C for 15 minutes. The sample was centrifuged at maximum for 15 minutes in a bench centrifuge and the supernatant discarded. The pellet was washed twice with ice-cold 70% v/v ethanol and once with ice-cold ethanol and allowed to air dry. The pellet was resuspended in 60 $\mu$L sterile milliQ water by rocking at 37 °C for 1 hour. This was used in subsequent PCR reactions as template.

Amplification.

A probe/primer set (MWG Biotech) was used based upon a clinical diagnostic PCR used to detect the *Chlamydia trachomatis ompA* gene (Personal communication, Prof Paul Wallace). The primers were designed to amplify a 100 bp region of the gene from base 501 to base 600 (based upon GenBank sequence AY535172) the probe anneals to bases 596 to 535. The sequences of the primers and probe used are detailed below 5' to 3':

| | |
|---|---|
| Primer 1: | cacttratattaccgcaggaacag |
| Primer 2: | gctaaacttgctgccactcat |
| Probe: | Biotin-agaggcatccttagtccctgtcgcagc-R6G |

PCR was carried out using hotstart Nova*Taq* polymerase (Novagen), 25 pmol of each primer, between 100pmol and 0.1pmol of probe, 2 $\mu$L template and 3 mM $MgSO_4$. The total volume of reactions was 50 $\mu$L and these were carried out under mineral oil. PCR was performed over 30 amplification cycles using the following parameters:

| | |
|---|---|
| 1 cycle | 94°C for 10 mins |
| 30 cycles | 94°C for 20s, 58°C for 20s, and 72°C for 20s |
| 1 cycle | 72°C for 2 mins. |

**Sample processing with streptavadin beads**

Streptavadin paramagnetic beads were obtained at 4 $mg.mL^{-1}$ from New England Biolabs. The beads have a binding capacity of 500 $pmol.mg^{-1}$ of beads for biotinylatyed oligonucleotides. As the maximum amount of biotinylatyed probe in any PCR reaction was 100 pmol, 0.2 mg (50 $\mu$L) of beads are required per reaction.

Preparing Streptavadin beads for use.

The beads were resuspended by gentle vortexing. The required aliquot (50 $\mu$L multiplied by the number of PCR reactions to be assayed) was removed and placed in a sterile 1.5 mL tube. The beads were placed in a magnetic separation rack for ≥ 2 minutes until all the beads had been sequestered. Keeping the tube in the rack, the supernatant was removed and discarded. The tube was removed from the rack and the beads resuspended in 2 volumes 2x B/W buffer, [2x Bind and wash buffer: 10 mM Tris-HCl pH 7.5, 1 mM EDTA pH 7.5, 2 M NaCl]. The tube was returned to the separation rack and incubated for ≥ 2 minutes. The washing process was repeated twice. The beads were resuspended in 2x B/W so that their final volume was the same as the starting volume.

Sample processing

50 μL of prepared beads was added to 50 μL of PCR reaction and mixed gently before being incubated for ≥15 minutes at room temperature with occasional mixing. The tube was placed in the separation rack so that the magnet was above the layer of mineral oil and incubated at room temperature for ≥ 2 minutes or until the beads had migrated above the mineral oil. The tube was kept on the rack while the supernatant was removed to a fresh tube. This supernatant was then used for all subsequent SERRS analysis.

SERRS Detection

Preparation of silver nanoparticles.

A colloidal suspension of citrate-reduced silver nanoparticles was prepared using a modified Lee and Meisel procedure.

Instrumentation.

The following Raman instrumentation was used: a Renishaw model 100 probe system with a 514.5-nm argon ion laser, utilizing a 20x objective to focus the laser beam into a 1-cm plastic cuvette containing the sample.

Sample Preparation.

All samples were prepared for SERRS analysis using the following amounts of reagents: 10 μL of analyte, 10 μL of spermine, 250 μL of water, and 250 μL of citrate-reduced silver nanoparticles.

Agarose gel electrophoresis

DNA was visualised on horizontal, neutral, 2.5 % (w/v) agarose gels. Gels were routinely prepared and run in 1 x TBE buffer (Sigma). 100 bp marker (Novagen) and Hyper V (Bioline) markers were used as size standards. Large 16·5 x 23cm (200 mL) gels were used to ensure good separation. DNA samples were mixed with 1/10 volume of 10x Bluejuice loading buffer (Sigma) before loading onto the gel. Gels were stained in a 0.5 $\mu g.mL^{-1}$ solution of ethidium bromide dissolved in 1x TBE for 10-20 minutes.

**Results and discussion.**

This technique is an adaptation of *Taq*-man using a primer set to amplify a specific region of *ompA* from *C. trachomatis* with the concomitant digestion of a duel labelled probe.

In *Taq*-man there are two primers and a dual-labelled probe that anneals between them. The probe carries a quencher at one end and a fluorescent dye at the other. While the probe is intact the quencher comes into close enough proximity to the fluorescent label to prevent emission. During the PCR amplification the polymerase's 5' to 3' nuclease activity digests the probe as it extends the primers, effectively separating the quencher from the fluorescent label and allowing detection.

In this example the probe was labelled with biotin and rhodamine. During amplification a proportional amount of the probe is destroyed releasing biotin and rhodamine. Both are still attached to a few nucleotides but no longer physically associated with each other. After PCR there is a mixture of unreacted (intact) probe with both biotin and rhodamine attached, free biotin and free rhodamine.

The streptavadin beads, in an almost irreversible interaction between streptavadin and biotin, readily capture the intact probe and free biotin leaving only the free rhodamine in the supernatant. This process sequesters any unreacted probe and removes it from the subsequent SERRS analysis so that a rhodamine signal indicates a positive test for C. *trachomatis.*

Due to the extremely sensitive nature of SERRS the amount of probe used may be minimised if desired to reduce any background and to reduce cost. We investigated a range of probe concentrations to see which might be the most effective, from 100 pmol to 0.1 pmol. The experiment was carried out in duplicate with two sets of positive and two sets of negative PCR carried out, (negative PCR lacked C. *trachomatis* DNA). A 10 μL aliquot of each PCR was electrophoresed on a 2.5% (w/v) TBE agarose gel to check for the presence of product, Figure 18. All of the positive reactions amplified well and none of the negative reactions showed any product.

Figure 18 shows a test of amplification by PCR: a 2.5% (w/v) agarose TBE gel showing two positive replicates (lanes 2 to 9) and two negative replicates (lanes 12 to 19) for the probe dilution range 100pmol probe, 10pmol, 1 pmol

and 0.1pmol. Lane 1), 100bp markers, 2) 100a, 3) 100b, 4) 10a, 5) 10b, 6) 1 a, 7) 1 b, 8) 0.1 a, 9) 0.1 b, 10) 100bp marker, 11) Hyper V markers, 12) 100a, 13) 100b, 14) 10a, 15) 10b, 16) 1 a, 17) 1 b, 18) 0.1 a, 19) 0.1 b, 20) 100bp marker

The PCR reactions were made back up to 50 $\mu$L and processed using streptavadin beads to separate the intact and digested probe. The supernatant was then used for SERRS analysis.

The SERRS analysis showed that the positive reactions gave a strong SERRS signal and that although there were also signals from the PCR negative samples, these were many times less intense than the positive controls. See Fig 19 & 20. Fig. 19 shows the spectra for the probe dilution experiment with the graph showing the two positive series (red and green) and the two negative series (blue and black) of samples analysed by SERRS. Probe concentration increases from left to right. Fig 20 shows difference in spectra between positive and negative samples using 100 pmol of probe per reaction.

**ExoSERRS Bead Assay**

A so-called exoSERRS bead assay is depicted in Figure 21. At the beginning of the scheme is depicted, uppermost, a single-stranded target DNA and a short capture probe that is biotinylated at the 3' end (depicted as the "B" inside the small pale grey rectangle that has been bound to streptavadin-coated magnetic bead shown as the large grey circle. After binding of the 3'-biotinylated capture probe to the streptavadin-coated magnetic beads, unbound probe is washed away. In the first step shown, the target DNA is hybridised to the immobilised capture probe. Unbound target DNA may be washed away. A 5'-phosphate label of a nucleotide comprise a SERRS active dye (the pale grey circle shown towards the 5' end (in this case TAMRA) and also, optionally a 3'-label quencher (in this case BHQ2 is allowed to hybridise to the captured target DNA. Finally the resultant double-stranded DNA comprising the target DNA, the capture probe and the dye-labelled probe is exposed to the action of lambda exonuclease, which degrades the hybridised dye-labelled probe DNA to mononucleotides, freeing the dye whereby to allow detection by SERRS.

The assay depicted in Figure 21, utilizing the strong biotin-streptavidin interaction, uses a short capture probe, a full length target and a dye label probe. The split probe assay employs the three above synthetic oligonucleotides, of the following composition:

**Capture Probe**

3' TCT CCG TAG GAA — 3' dT is biotinylated

**Dye Label Probe**

3' TCA GGG ACA GCG XCG — 3' dT modified with BHQ2; X is dT modified with TAMRA

**Target Complement**

5' AGA GGC ATC CTT AGT CCC TGT CGC AGC

**Experimental**

1. The capture probe is bound to streptavidin coated magnetic beads via the 3' biotin modification in 1 x bind and wash (B/W) buffer. Unbound probe is washed away with B/W buffer whilst the bound probe is separated by magnetisation.
2. The target complement sequence is hybridized to the bound capture probe using a heating program (90°C for 10 mins, 20°C for 5 mins). The unbound target is again washed away using 1 x B/W, for 3+ washes.
3. The dye label probe is then hybridized to the preformed capture probe / target duplex using the previous heating conditions. Once more, unbound probe is washed away as above.
4. The split probe complex is then exposed to $\lambda$ exonuclease and exonuclease reaction buffer. Digestion is carried out at 37°C for 30 mins followed by an enzyme inactivation step of 75°C for 15 mins.
5. The complex is transferred to a 96 well microtitre plate and silver colloid, aggregating agent and water added in preparation for SERRS.
6. SERRS is taken immediately using a Renishaw inVia Raman at 514.5nm, 100% power, x20 objective, 10s and 3 accumulations.
7. Fluorescence measurements are also possible.

**Results**

Figure 22 illustrates the SERRS response of a complete run through of the assay versus a run through minus the dye labelled probe. The shape of the TAMRA response is highly visible. The absence of peaks in the control is encouraging.

**Synthesis of a SERRS active Probe**

A SERRS active probe was synthesised which consisted of a probe sequence containing a SERRS label that is inactive, i.e. giving no SERRS signal, when it is in the form of the probe. Once this sequence hybridises to the target sequence the probe will remain inactive until after the action of λ or other exonuclease, which releases the SERRS label whereby to make it SERRS active.

SERRS active probes may be made by generating an oligonucleotide with a 5' phosphate, the probe sequence and then a 3' phosphate linkage onto the dye which will become SERRS active after the enzymatic cleavage. The 3' phosphate linkage permits direct attachment to hydroxyquinoline azo dyes which have been shown to be good substrates for alkaline phosphatase in going from an 'off' to an 'on' situation when using SERRS detection (F.M. Campbell et al., Analyst, 2008, DOI 10.1039/B8087A). In this approach, a hydroxyquinoline azo dye is added to the 3' end using reversed phosphoramidite synthesis of oligonucleotides i.e. synthesis from 5' to 3' direction (normal oligonucleotide synthesis is from 3'-5'). This allows addition of the hydroxyquinoline azo dyes as a phosphoramidite during standard solid phase synthesis. By changing the additional aromatic component of the azo dye different dyes can be used with different sequences, each with a different SERRS signal. This thus enables multiplexing

**8-Hydroxyquinoline-derived dye synthesis**

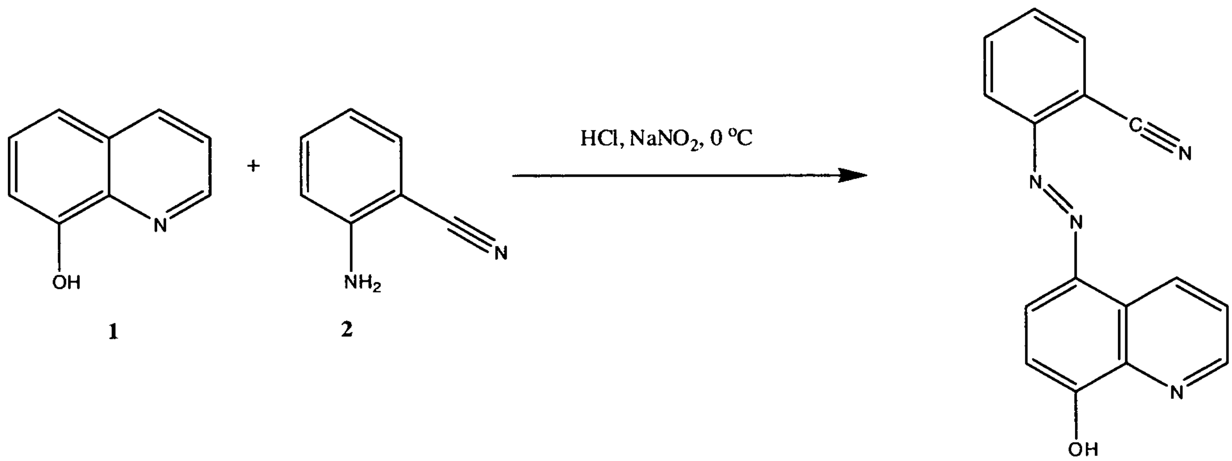


1 2 3

The 8-hydroxyquinoline-derived dye (3) was prepared by diazotization of *o*-aminobenzonitrile followed by coupling with 8-hydroxyquinoline. To purify the dye it was first acetylated so that it could be separated from starting materials by flash column chromatography, and de-acetylated prior to conversion to phosphoramidite. Synthesis of the dye was confirmed by $^1$H, $^{13}$C NMR, mass spec.

*Experimental:*

*0*-Aminobenzonitrile (1 eq) was stirred in HCl (50 %, 10 ml) and gently heated for 10 min. The mixture was cooled to 0 °C in an ice bath, to which $NaNO_2$ (1.2 eq) in 2 ml of water (distilled) was added dropwise. The reaction was left to stir for 30 min at 0 °C, yielding a pale yellow solution. Separately, 8-hydroxyquinoline (1 eq) was dissolved in MeOH (30 ml) to which NaOH (10 %, 100 ml) was added. The 8-hydroxyquinoline solution was added dropwise to the diazonium salt solution over 15 min and stirred overnight.

The reaction was neutralized by the addition of HCl (50 %) and the resulting precipitate collected by filtration and dried overnight. The precipitate was dissolved in acetic anhydride (100 ml) with a catalytic amount of 4-dimethylaminopyridine. TLC analysis showed complete reaction. The mixture was poured into 1600 ml of ice/water and left for 3 h. Excess water was poured off and more ice added to red oil/water mixture causing precipitation. The precipitate was collected and dried and purified by flash column chromatography (silica; eluent: DCM - 1 % MeOH/DCM) yielding the deacetylated target product, 3 in 38 % yield. $\delta_H$(400 MHz; DMSO) 3.31 (1H, br, s, OH), 7.29-7.31 (1 H, d, *J* 8.0, ArH), 7.67-7.71 (1H, td, ArH), 7.78-7.81 (1H, q, *J* 4.0, ArH), 7.87-7.91 (1H, td, ArH), 8.06-8.12 (3H, m, ArH x 3), 9.00 - 9.02 (1H, dd, ArH), 9.36-9.38 (1 H, dd, ArH).

**SERRS of 8-hydroxyquinoline-derived dye, 3**

SERRS activity of dye 3 was confirmed by analysis of a solution (0.1 μM) using 1 % poly-L-lysine as aggregating agent. 514 nm laser source, 1 s acquisition, 100 % power, 1400 $cm^{-1}$. The SERRS spectrum is shown in Figure 23.

**8-Hydroxyquinoline-derived dye DNA modification**

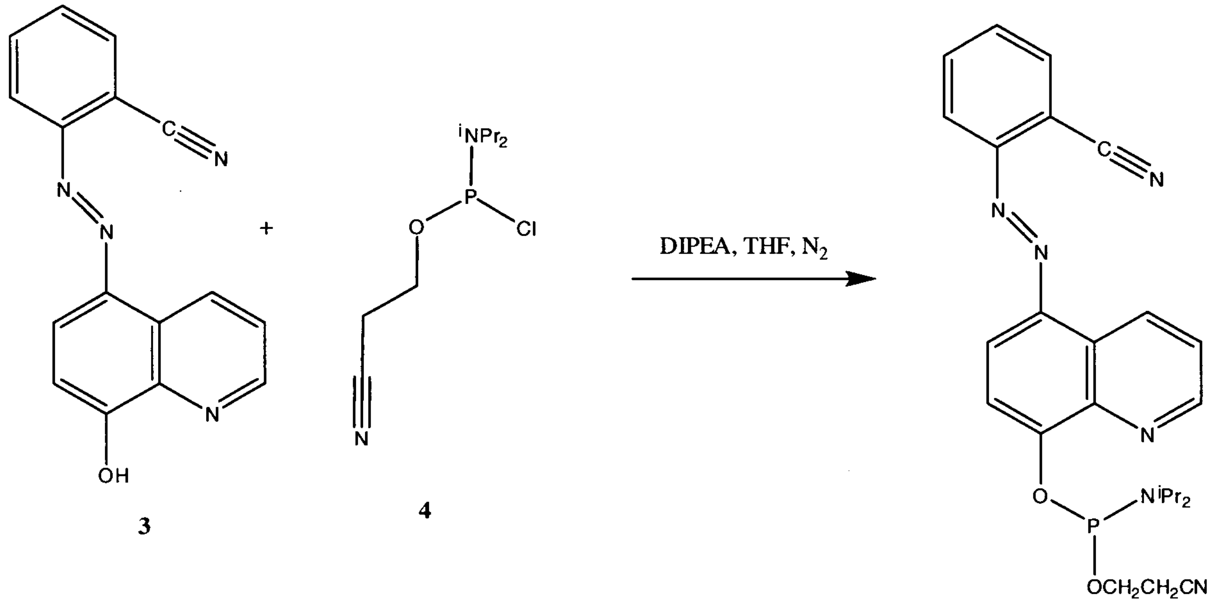


3

4

5

Phosphitylation of 8-hydroxyquinoline-derived dye, **3,** was carried out with chlorophosphitylating reagent, **4** to yield phosphoramidite, **5.** Synthesis of a $P^{III}$ phosphoramidite was confirmed by $^{31}P$ NMR. Phosphoramidite **5** was used to generate 3'-dye-labelled probe sequences using reverse base synthesis, incorporating a phosphate at the 5'-end by use of a phosphate CPG column.

## Claims

1. A method for use in the detection of a target nucleic acid comprising the steps of:

   (i) contacting a single-stranded probe nucleic acid with a sample of interest under conditions effective to generate a probe/target nucleic acid duplex by specific hybridisation of said probe nucleic acid to a target nucleic acid, if said target nucleic acid is present;
   (ii) contacting any probe/target nucleic acid duplex with an exonuclease to effect digestion of the duplex and release of a label molecule from the duplex; and
   (iii) detecting the label by Raman spectroscopy by detecting a detectable change in the Raman spectrum of the label upon its release from the duplex, so as to detect the target nucleic acid, if present.

2. The method of claim 1 wherein said exonuclease has no oligonucleotide-synthesising ability; and/or wherein said probe nucleic acid has a 5'-phosphate group and said exonuclease is lambda exonuclease.

3. The method of claim 1 or claim 2 wherein an excess of said probe nucleic acid is contacted with the sample of interest in step (i) such that digestion of a duplex in step (ii) recycles the target nucleic acid one or more times thereby allowing further probe molecules to specifically hybridise to the target forming additional probe/target nucleic acid duplexes that are digested to release further label molecules.

4. The method of any one preceding claim wherein the quantity of any target nucleic acid in said sample of interest is determined with reference to the magnitude of said change.

5. The method of any one preceding claim wherein the probe nucleic acid and/or the target nucleic acid is a DNA.

6. The method of any one preceding claim wherein said target nucleic acid is a double-stranded nucleic acid or a single-stranded nucleic acid.

7. The method of any one preceding claim wherein the label is only capable of being detected by way of Raman spectroscopy when released from the duplex nucleic acid by way of digestion of the probe/target nucleic acid duplex.

8. The method of any one preceding claim wherein said label is a SE(R)RS active dye; and/or wherein the label is a fluorophore.

9. The method of any one preceding claim wherein said probe comprises a fluorophore and quencher that quenches the fluorophore prior to said digestion; and/or wherein said detecting by Raman spectroscopy is supplemented by detection based on plasmonics or fluorescence.

10. The method of any one preceding claim wherein the probe sequence is attached to a SE(R)RS active substrate.

11. The method of any one preceding claim wherein the probe nucleic acid optionally comprises from about 20 to 30 nucleotides.

12. The method of claim 11 wherein the label is contacted with said probe nucleic acid and said sample of interest in step (i), which label can intercalate with any of said probe/target nucleic acid duplex if formed.

13. The method of claim 11 wherein the label is attached, bonded or otherwise associated with the probe nucleic acid, for example wherein the probe nucleic acid nucleic is bonded to the label.

14. The method of any one of claims 1 to 9 wherein said contacting comprises contacting with a first probe nucleic acid and a second probe nucleic acid, wherein said first probe nucleic acid comprises a sequence of nucleic acid complementary to a portion of said target nucleic acid, and a capturable moiety, which permits capture of any duplex resultant from hybridisation of first probe nucleic acid to target nucleic acid, and wherein said second probe comprises a sequence of nucleic acid complementary to a portion of said target nucleic acid other than that to which said first probe nucleic acid is complementary and said label.

15. The method of claim 14 wherein the duplex formed by contacting said sample of interest with said first and said second probe nucleic acids is isolated from other material not part of said duplex prior to said detecting after said contacting.

16. The method of any one of claims 1 to 9 wherein said contacting comprises contacting with a first probe nucleic acid and a second probe nucleic acid, wherein said first probe nucleic acid comprises a sequence of nucleic acid complementary to a portion of said target nucleic acid, and a sequence of nucleic acid complementary to a portion of said second probe nucleic acid.

17. The method of claim 16 wherein said contacting further comprises contacting with a capture nucleic acid, which capture nucleic acid is complementary to a portion of said target nucleic acid to which said first probe nucleic acid is not complementary and bound to a capturable moiety which permits capture of any capturable nucleic acid complex, which capturable nucleic acid complex may be formed upon said contacting when said target nucleic acid is present, such as wherein said capturable nucleic acid complex is isolated from other material not part of said duplex prior to said detecting after said contacting.

18. The method of any one of claims 14 to 17 wherein the label is attached, bonded or otherwise associated with the second probe nucleic acid, for example wherein the second probe nucleic acid nucleic is bonded to the label; and/or wherein the first and second probe nucleic acids each comprise from about 20 to about 30 nucleotides.

19. A method for simultaneously detecting a plurality of different target nucleic acids in a sample of interest comprising simultaneously effecting a plurality of methods as defined in any one of claims 1 to 18 in which a different label is

used for detecting each of said target nucleic acids.

## Patentansprüche

1. Verfahren zur Anwendung beim Nachweis einer Ziel-Nukleinsäure, umfassend die Schritte:

   (i) In-Kontakt-Bringen einer einzelsträngigen Sonden-Nukleinsäure mit einer Probe von Interesse unter Bedingungen, die effektiv sind zur Erzeugung eines Sonden/Ziel-Nukleinsäure-Duplex durch spezifische Hybridisierung der Sonden-Nukleinsäure an eine Ziel-Nukleinsäure, falls die Ziel-Nukleinsäure vorhanden ist;
   (ii) In-Kontakt-Bringen von jeglichem Sonden/Ziel-Nukleinsäure-Duplex mit einer Exonuklease, um einen Verdau des Duplex und eine Freisetzung eines Markierungsmoleküls aus dem Duplex zu bewirken; und
   (iii) Nachweisen der Markierung mittels Raman-Spektroskopie durch Nachweisen einer nachweisbaren Veränderung im Raman-Spektrum der Markierung bei ihrer Freisetzung aus dem Duplex, um die Ziel-Nukleinsäure, falls vorhanden, nachzuweisen.

2. Verfahren nach Anspruch 1, wobei die Exonuklease keine Oligonukleotidsynthetisierende Fähigkeit aufweist; und/oder wobei die Sonden-Nukleinsäure eine 5'-Phosphatgruppe aufweist und die Exonuklease Lambda-Exonuklease ist.

3. Verfahren nach Anspruch 1 oder Anspruch 2, wobei ein Überschuss der Sonden-Nukleinsäure mit der Probe von Interesse in Schritt (i) so in Kontakt gebracht wird, dass der Verdau eines Duplex in Schritt (ii) die Ziel-Nukleinsäure ein oder mehrere Male rezykliert, wodurch ermöglicht wird, dass weitere Sondenmoleküle spezifisch an den Ziel bildenden, zusätzlichen Sonden/Ziel-Nukleinsäure-Duplexen hybridisieren, die verdaut werden, um weitere Markierungsmoleküle freizusetzen.

4. Verfahren nach einem der vorstehenden Ansprüche, wobei die Menge jeglicher Ziel-Nukleinsäure in der Probe von Interesse bestimmt wird in Bezug auf die Größe der Veränderung.

5. Verfahren nach einem der vorstehenden Ansprüche, wobei die Sonden-Nukleinsäure und/oder die Ziel-Nukleinsäure eine DNA ist.

6. Verfahren nach einem der vorstehenden Ansprüche, wobei die Ziel-Nukleinsäure eine doppelsträngige Nukleinsäure oder eine einzelsträngige Nukleinsäure ist.

7. Verfahren nach einem der vorstehenden Ansprüche, wobei sich die Markierung lediglich durch Raman-Spektroskopie nachweisen lässt, wenn sie aus der Duplex-Nukleinsäure durch Verdau des Sonden/Ziel-Nukleinsäure-Duplex freigesetzt wird.

8. Verfahren nach einem der vorstehenden Ansprüche, wobei die Markierung ein SE(R)RS-aktiver Farbstoff ist; und/oder wobei die Markierung ein Fluorophor ist.

9. Verfahren nach einem der vorstehenden Ansprüche, wobei die Sonde einen Fluorophor und einen Löscher umfasst, der den Fluorophor vor dem Verdau löscht; und/oder wobei das Nachweisen durch Raman-Spektroskopie ergänzt wird durch einen Nachweis auf Grundlage von Plasmonik oder Fluoreszenz.

10. Verfahren nach einem der vorstehenden Ansprüche, wobei die Sondensequenz an ein SE(R)RS-aktives Substrat angefügt ist.

11. Verfahren nach einem der vorstehenden Ansprüche, wobei die Sonden-Nukleinsäure gegebenenfalls etwa 20 bis 30 Nukleotide umfasst.

12. Verfahren nach Anspruch 11, wobei die Markierung in Kontakt gebracht wird mit der Sonden-Nukleinsäure und der Probe von Interesse in Schritt (i), welche Markierung mit jeglichem von dem Sonden/Ziel-Nukleinsäure-Duplex, falls gebildet, interkalieren kann.

13. Verfahren nach Anspruch 11, wobei die Markierung angefügt, gebunden oder in sonstiger Weise mit der Sonden-Nukleinsäure assoziiert wird, wobei beispielsweise die Sonden-Nukleinsäure an die Markierung gebunden wird.

**14.** Verfahren nach einem der Ansprüche 1 bis 9, wobei das In-Kontakt-Bringen In-Kontakt-Bringen mit einer ersten Sonden-Nukleinsäure und einer zweiten Sonden-Nukleinsäure umfasst, wobei die erste Sonden-Nukleinsäure eine Nukleinsäuresequenz, die komplementär ist zu einem Teil der Ziel-Nukleinsäure, und einen fangbaren Anteil umfasst, welcher ein Fangen von jeglichem Duplex gestattet, der aus Hybridisierung der ersten Sonden-Nukleinsäure an Ziel-Nukleinsäure resultiert, und wobei die zweite Sonde eine Nukleinsäuresequenz, die komplementär ist zu einem Teil der Ziel-Nukleinsäure, jenen ausgenommen, zu dem die erste Sonden-Nukleinsäure komplementär ist, und die Markierung umfasst.

**15.** Verfahren nach Anspruch 14, wobei der Duplex, der durch In-Kontakt-Bringen der Probe von Interesse mit der ersten und der zweiten Sonden-Nukleinsäure gebildet wird, von sonstigem Material, das nicht Teil des Duplex ist, isoliert wird vor dem Nachweisen nach dem In-Kontakt-Bringen.

**16.** Verfahren nach einem der Ansprüche 1 bis 9, wobei das In-Kontakt-Bringen In-Kontakt-Bringen mit einer ersten Sonden-Nukleinsäure und einer zweiten Sonden-Nukleinsäure umfasst, wobei die erste Sonden-Nukleinsäure eine Nukleinsäuresequenz, komplementär zu einem Teil der Ziel-Nukleinsäure, und eine Nukleinsäuresequenz, komplementär zu einem Teil der zweiten Sonden-Nukleinsäure, umfasst.

**17.** Verfahren nach Anspruch 16, wobei das In-Kontakt-Bringen weiterhin In-Kontakt-Bringen mit einer Fang-Nukleinsäure umfasst, welche Fang-Nukleinsäure komplementär ist zu einem Teil der Ziel-Nukleinsäure, zu dem die erste Sonden-Nukleinsäure nicht komplementär ist, und gebunden an einen fangbaren Anteil, der ein Fangen von jeglichem fangbaren Nukleinsäurekomplex gestattet, welcher fangbare Nukleinsäurekomplex beim In-Kontakt-Bringen gebildet werden kann, wenn die Ziel-Nukleinsäure vorhanden ist, wobei z. B. der fangbare Nukleinsäurekomplex von sonstigem Material, das nicht Teil des Duplex ist, isoliert wird vor dem Nachweisen nach dem In-Kontakt-Bringen.

**18.** Verfahren nach einem der Ansprüche 14 bis 17, wobei die Markierung angefügt, gebunden oder in sonstiger Weise mit der zweiten Sonden-Nukleinsäure assoziiert wird, wobei zum Beispiel die zweite Sonden-Nukleinsäure an die Markierung gebunden wird; und/oder wobei die erste und die zweite Sonden-Nukleinsäure jeweils etwa 20 bis etwa 30 Nukleotide umfassen.

**19.** Verfahren zum simultanen Nachweisen mehrerer verschiedener Ziel-Nukleinsäuren in einer Probe von Interesse, umfassend simultanes Ausführen mehrerer Verfahren gemäß Definition in einem der Ansprüche 1 bis 18, wobei zum Nachweisen einer jeden der Ziel-Nukleinsäuren eine andere Markierung verwendet wird.

## Revendications

**1.** Procédé pour une utilisation dans la détection d'un acide nucléique cible comprenant les étapes:

(i) de mise en contact d'un acide nucléique sonde à brin simple avec un échantillon intéressant dans des conditions efficaces pour générer un duplex d'acides nucléiques sonde/cible par une hybridation spécifique dudit acide nucléique sonde à un acide nucléique cible, si ledit acide nucléique cible est présent;
(ii) de mise en contact de tout duplex d'acides nucléiques sonde/cible avec une exonucléase pour effectuer une digestion du duplex et une libération d'une molécule de marquage à partir du duplex; et
(iii) de détection du marquage par une spectroscopie Raman par la détection d'un changement détectable dans le spectre Raman du marquage à la suite de sa libération à partir du duplex, de manière à détecter l'acide nucléique cible, s'il est présent.

**2.** Procédé selon la revendication 1, dans lequel ladite exonucléase n'a pas la capacité de synthèse d'oligonucléotides; et/ou dans lequel ledit acide nucléique sonde possède un groupe 5'-phosphate et ladite exonucléase est une exonucléase lambda.

**3.** Procédé selon la revendication 1 ou la revendication 2, dans lequel un excès dudit acide nucléique sonde est mis en contact avec l'échantillon intéressant dans l'étape (i) de sorte que la digestion d'un duplex dans l'étape (ii) recycle l'acide nucléique cible une ou plusieurs fois permettant ainsi à des molécules sondes supplémentaires de s'hybrider spécifiquement aux duplexes d'acides nucléiques sonde/cible supplémentaires formant une cible qui sont digérés pour libérer des molécules de marquage supplémentaires.

**4.** Procédé selon l'une quelconque des revendications précédentes, dans lequel la quantité de tout acide nucléique

cible dans ledit échantillon intéressant est déterminée en faisant référence à la grandeur dudit changement.

5. Procédé selon l'une quelconque des revendications précédentes, dans lequel l'acide nucléique sonde et/ou l'acide nucléique cible est un ADN.

6. Procédé selon l'une quelconque des revendications précédentes, dans lequel ledit acide nucléique cible est un acide nucléique à brin double ou un acide nucléique à brin simple.

7. Procédé selon l'une quelconque des revendications précédentes, dans lequel le marquage ne peut être détecté qu'au moyen d'une spectroscopie Raman lorsqu'il est libéré à partir du duplex d'acides nucléiques au moyen d'une digestion du duplex d'acides nucléiques sonde/cible.

8. Procédé selon l'une quelconque des revendications précédentes, dans lequel ledit marquage est un colorant actif SE(R)RS; et/ou dans lequel le marquage est un fluorophore.

9. Procédé selon l'une quelconque des revendications précédentes, dans lequel ladite sonde comprend un fluorophore et un extincteur qui éteint le fluorophore avant ladite digestion; et/ou dans lequel ladite détection par une spectroscopie Raman est complétée par une détection basée sur les plasmoniques ou la fluorescence.

10. Procédé selon l'une quelconque des revendications précédentes, dans lequel la séquence sonde est attachée à un substrat actif SE(R)RS.

11. Procédé selon l'une quelconque des revendications précédentes, dans lequel l'acide nucléique sonde comprend optionnellement d'environ 20 à 30 nucléotides.

12. Procédé selon la revendication 11, dans lequel le marquage est mis en contact avec ledit acide nucléique sonde et ledit échantillon intéressant dans l'étape (i), lequel marquage peut s'intercaler avec n'importe lequel dudit duplex d'acides nucléiques sonde/cible s'il est formé.

13. Procédé selon la revendication 11, dans lequel le marquage est attaché, lié ou associé autrement avec l'acide nucléique sonde, par exemple dans lequel l'acide nucléique sonde est lié au marquage.

14. Procédé selon l'une quelconque des revendications 1 à 9, dans lequel ladite mise en contact comprend une mise en contact avec un premier acide nucléique sonde et un deuxième acide nucléique sonde, dans lequel ledit premier acide nucléique sonde comprend une séquence d'acide nucléique complémentaire à une portion dudit acide nucléique cible, et une fraction capturable, qui permet la capture de tout duplex résultant d'une hybridation du premier acide nucléique sonde à l'acide nucléique cible, et dans lequel ladite deuxième sonde comprend une séquence d'acide nucléique complémentaire à une portion dudit acide nucléique cible autre que celle à laquelle ledit premier acide nucléique sonde est complémentaire et ledit marquage.

15. Procédé selon la revendication 14, dans lequel le duplex formé par la mise en contact dudit échantillon intéressant avec ledit premier et ledit deuxième acides nucléiques sondes est isolé à partir d'un autre matériau ne faisant pas partie dudit duplex avant ladite détection après ladite mise en contact.

16. Procédé selon l'une quelconque des revendications 1 à 9, dans lequel ladite mise en contact comprend une mise en contact avec un premier acide nucléique sonde et un deuxième acide nucléique sonde, dans lequel ledit premier acide nucléique sonde comprend une séquence d'acide nucléique complémentaire à une portion dudit acide nucléique cible, et une séquence d'acide nucléique complémentaire à une portion dudit deuxième acide nucléique sonde.

17. Procédé selon la revendication 16, dans lequel ladite mise en contact comprend en outre une mise en contact avec un acide nucléique capture, lequel acide nucléique capture est complémentaire à une portion dudit acide nucléique cible auquel ledit premier acide nucléique sonde n'est pas complémentaire et lié à une fraction capturable qui permet la capture de tout complexe d'acide nucléique capturable, lequel complexe d'acide nucléique capturable peut être formé à la suite de ladite mise en contact lorsque ledit acide nucléique cible est présent, comme dans lequel ledit complexe d'acide nucléique capturable est isolé à partir d'un autre matériau ne faisant pas partie dudit duplex avant ladite détection après ladite mise en contact.

**18.** Procédé selon l'une quelconque des revendications 14 à 17, dans lequel le marquage est attaché, lié ou associé autrement avec le deuxième acide nucléique sonde, par exemple dans lequel le deuxième acide nucléique sonde est lié au marquage; et/ou dans lequel les premier et deuxième acides nucléiques sondes comprennent chacun d'environ 20 à environ 30 nucléotides.

**19.** Procédé pour la détection simultanée d'une pluralité d'acides nucléiques cibles différents dans un échantillon intéressant consistant à effectuer simultanément une pluralité de procédés tels que définis dans l'une quelconque des revendications 1 à 18, dans lequel un marquage différent est utilisé pour la détection de chacun desdits acides nucléiques cibles.

Figure 1

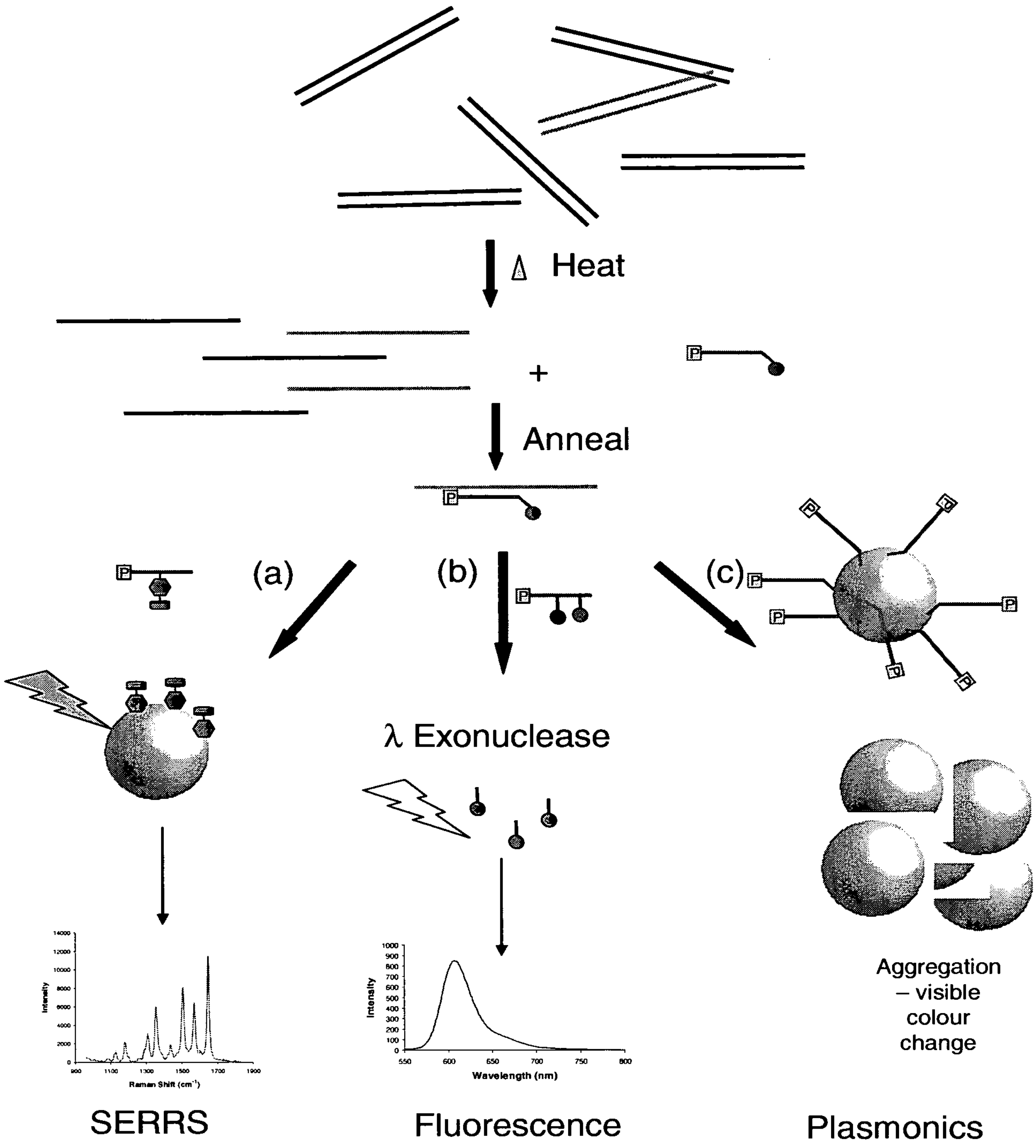
Heat
+
Anneal
(a)
(b)
(c)
λ Exonuclease
Intensity
Raman Shift (cm⁻¹)
Wavelength (nm)
Aggregation – visible colour change
SERRS
Fluorescence
Plasmonics

Figure 2

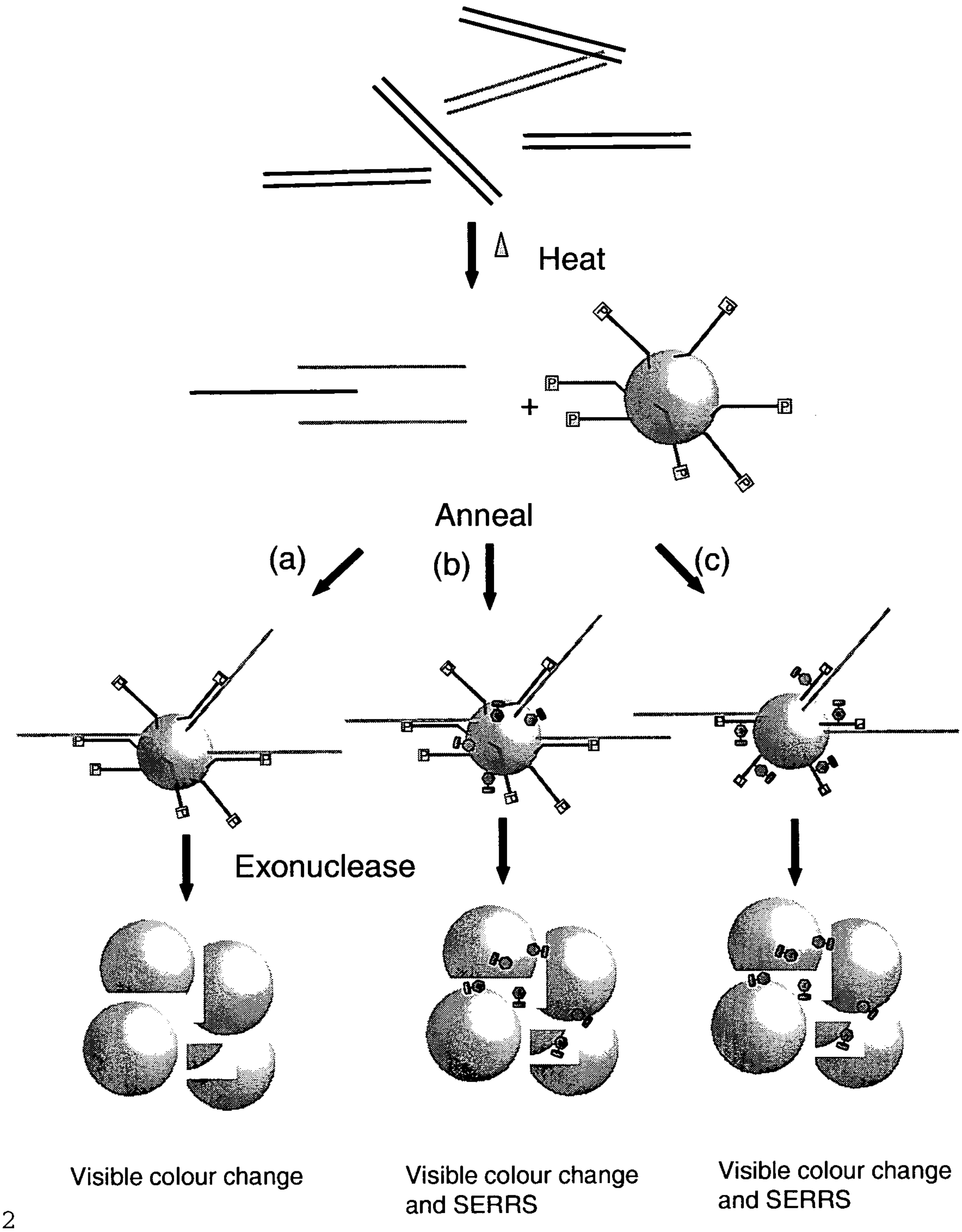
Heat
+
Anneal
(a)
(b)
(c)
Exonuclease
Visible colour change
Visible colour change
and SERRS
Visible colour change
and SERRS

2

Figure 3

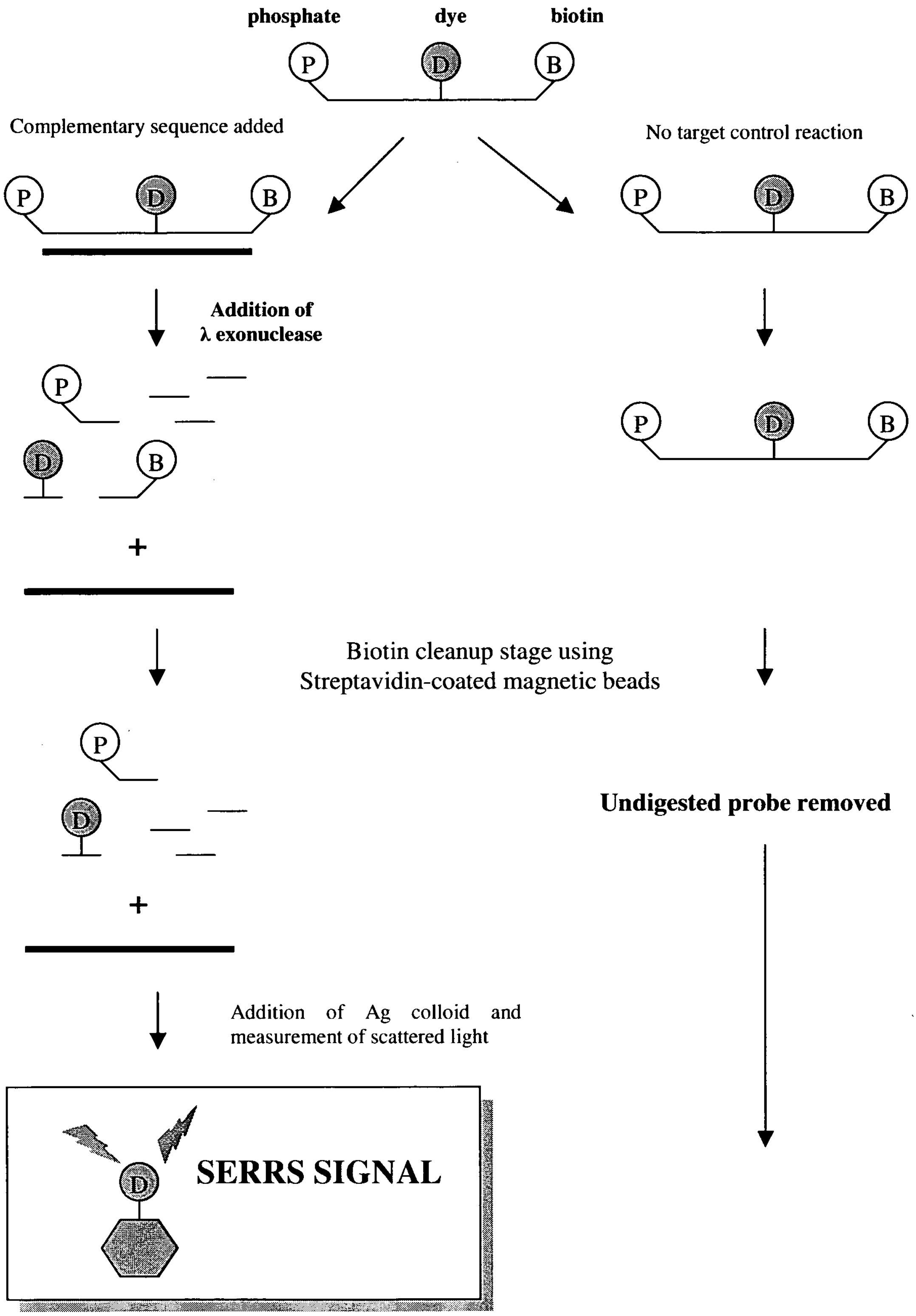
phosphate
dye
biotin
P
D
B
Complementary sequence added
No target control reaction
Addition of
λ exonuclease
+
Biotin cleanup stage using
Streptavidin-coated magnetic beads
Undigested probe removed
Addition of Ag colloid and
measurement of scattered light
SERRS SIGNAL

Figure 4

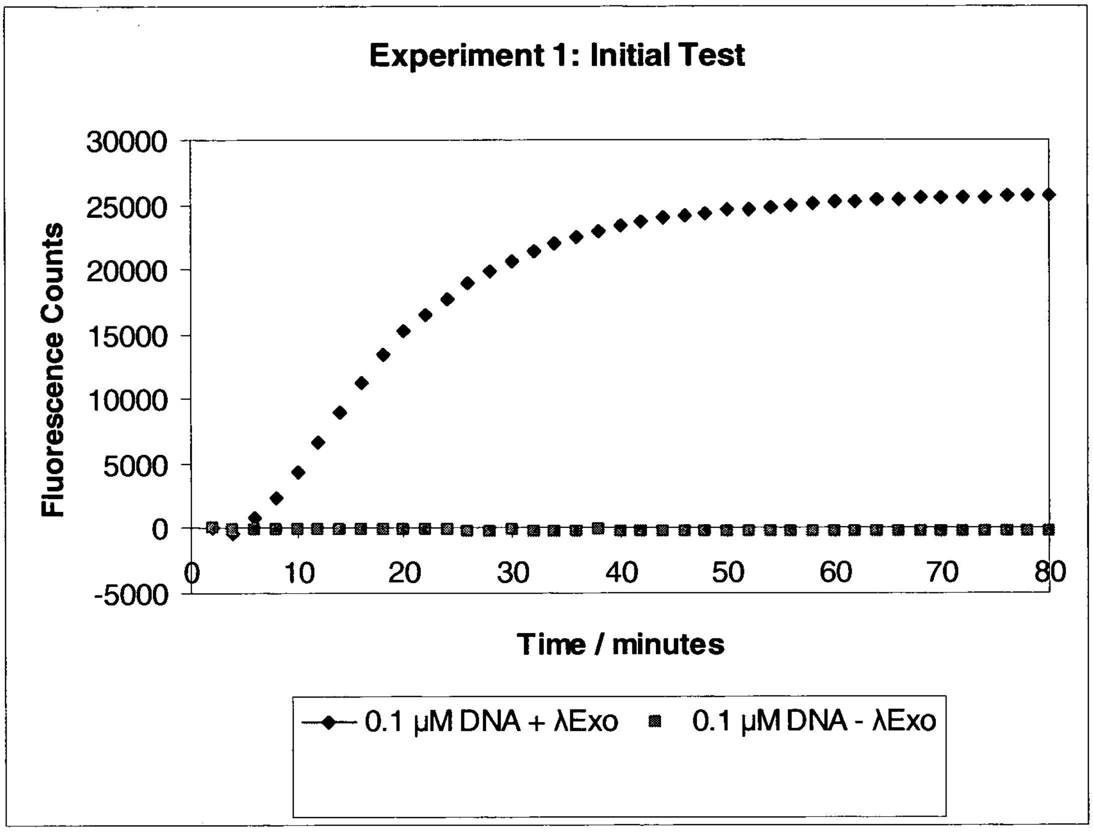
Experiment 1: Initial Test
Fluorescence Counts
30000
25000
20000
15000
10000
5000
0
-5000
0
10
20
30
40
50
60
70
80
Time / minutes
0.1 µM DNA + λExo
0.1 µM DNA - λExo

Figure 5

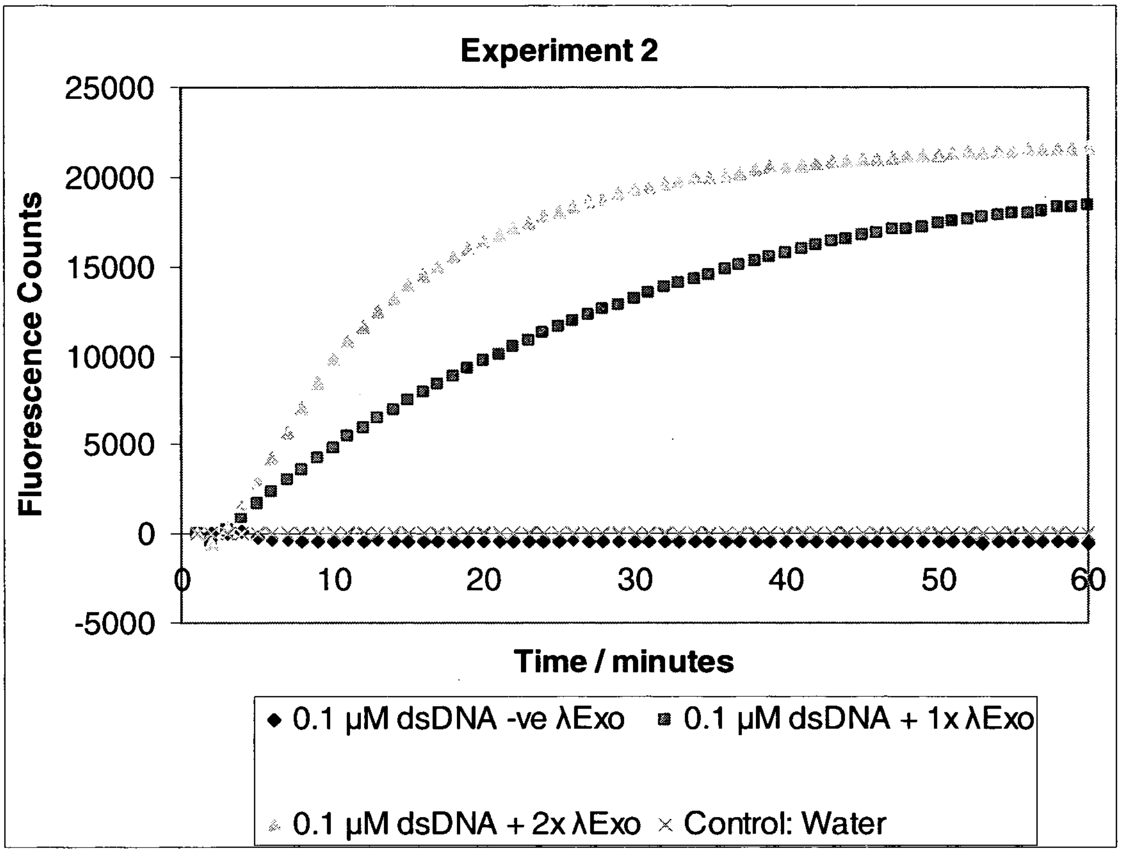
Experiment 2
25000
20000
15000
10000
5000
0
-5000
Fluorescence Counts
0
10
20
30
40
50
60
Time / minutes
0.1 μM dsDNA -ve λExo
0.1 μM dsDNA + 1x λExo
0.1 μM dsDNA + 2x λExo
Control: Water

Figure 6

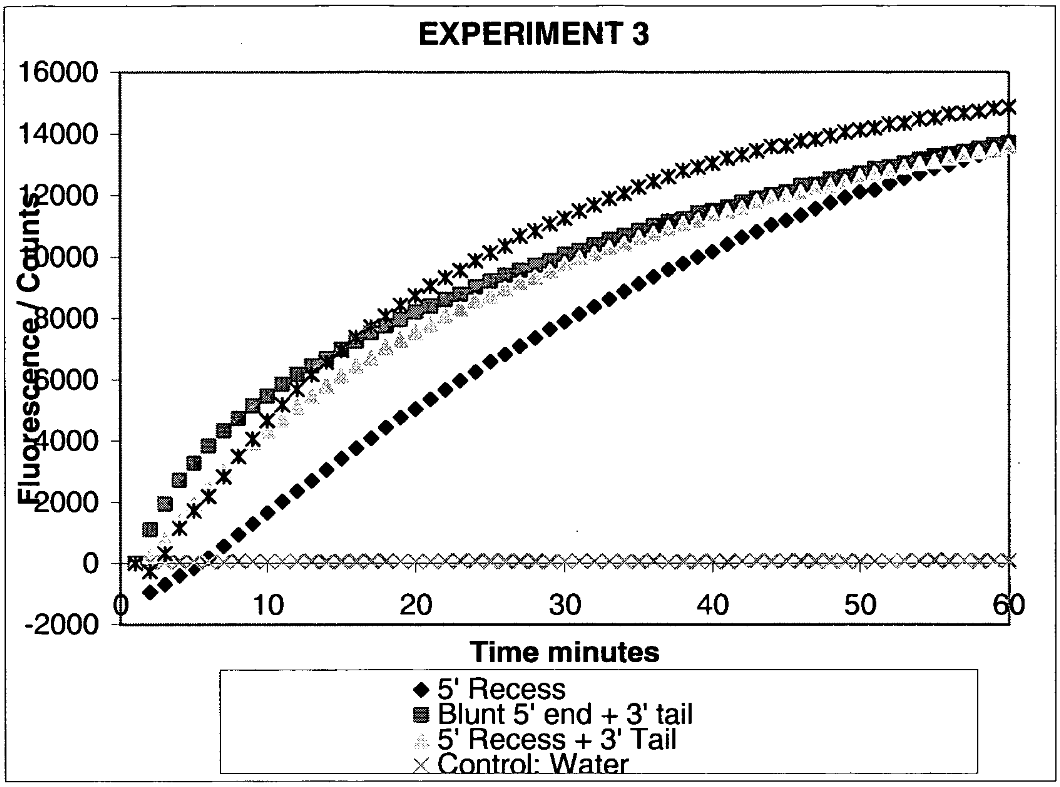


Figure 7

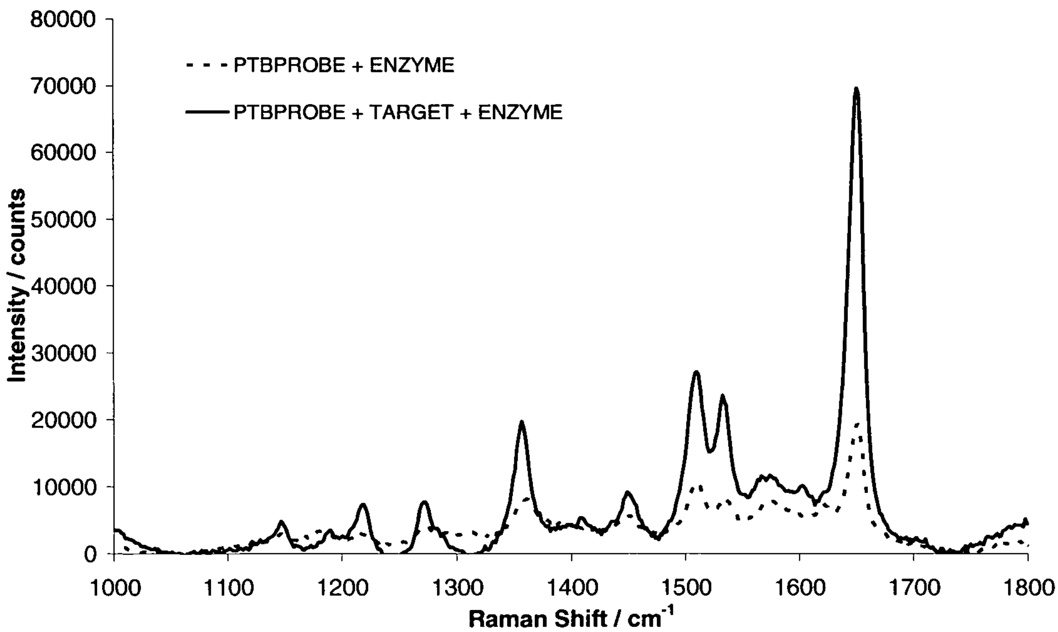

Figure 8

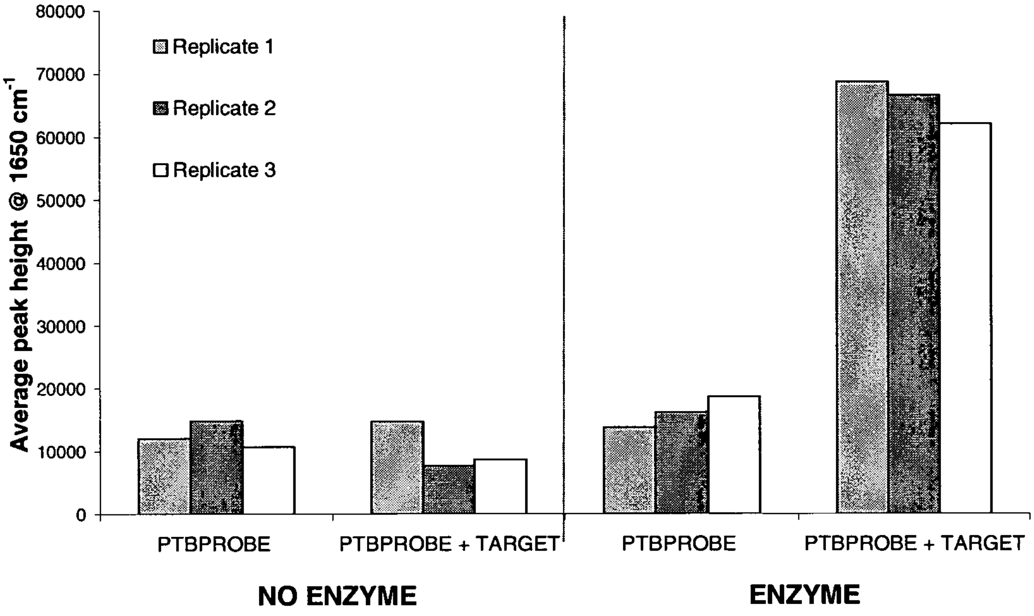


Figure 9

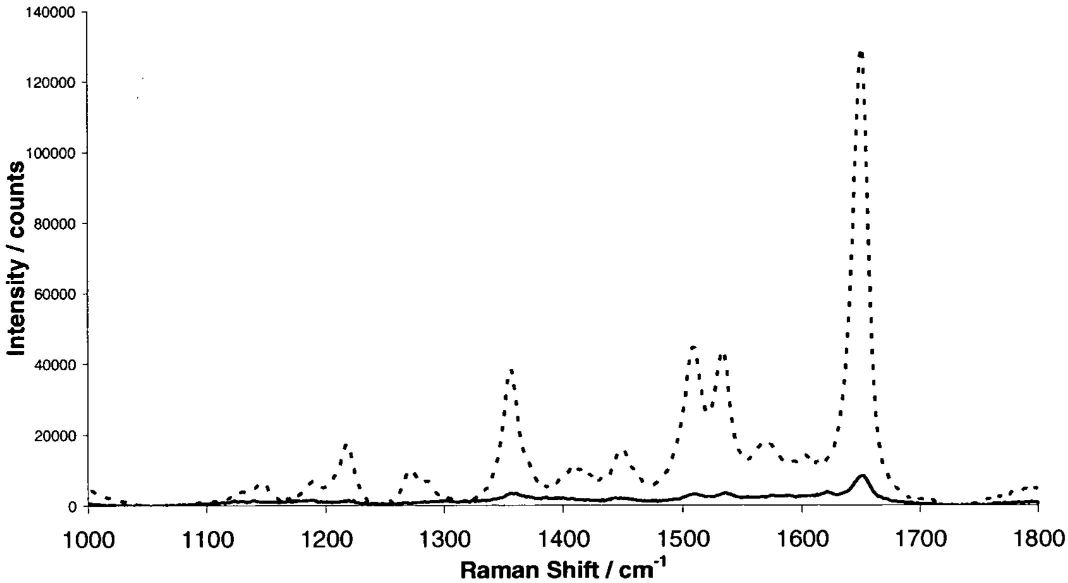


*Dotted line:* probe + target + enzyme

*Straight line:* probe + target (NEC, No Enzyme Control)

Figure 10

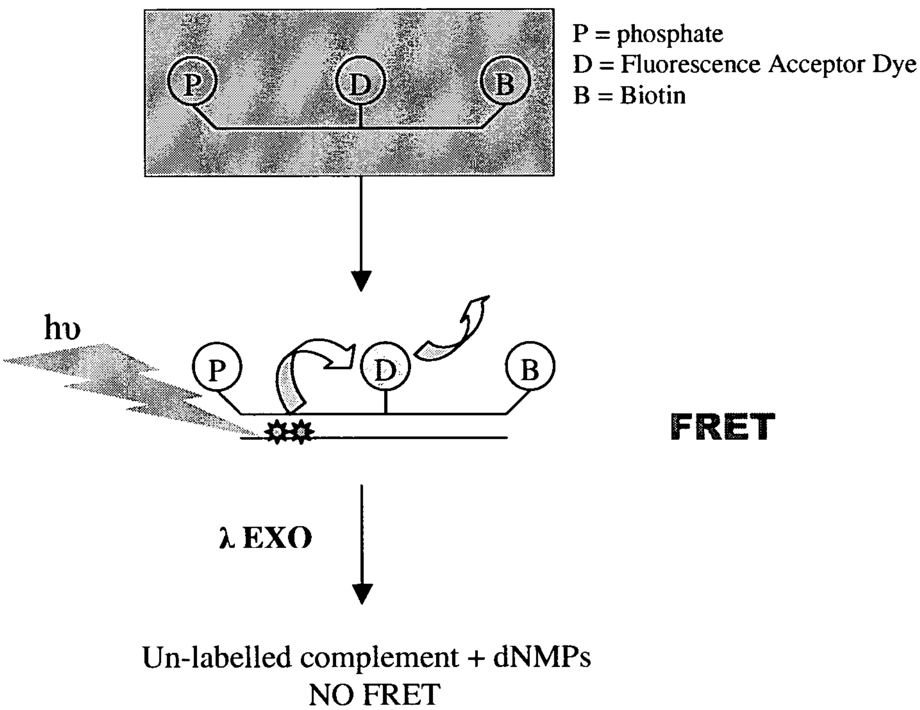
P = phosphate
D = Fluorescence Acceptor Dye
B = Biotin
P
D
B
hυ
FRET
λ EXO
Un-labelled complement + dNMPs
NO FRET

Figure 11

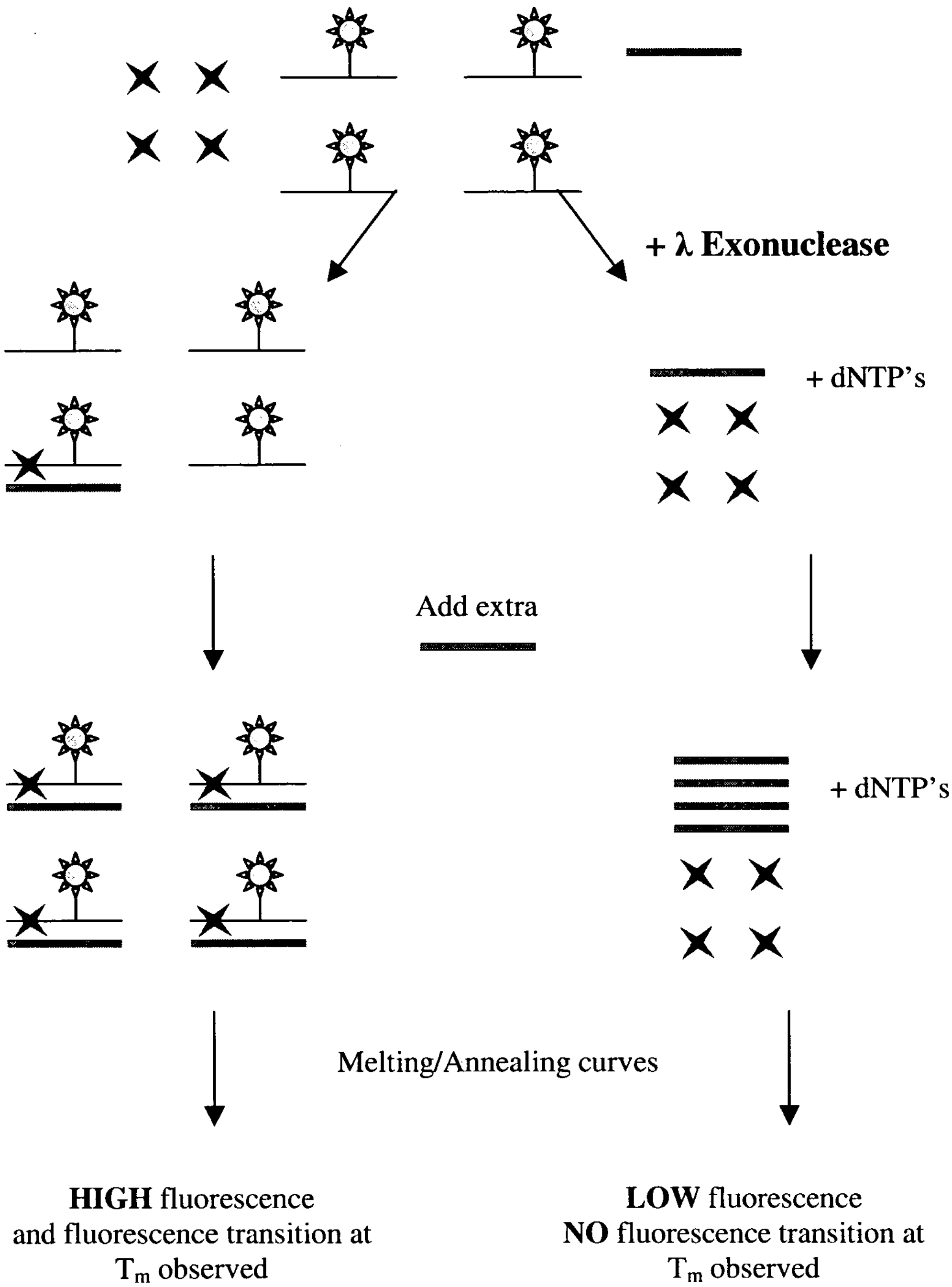
+ λ Exonuclease
+ dNTP's
Add extra
+ dNTP's
Melting/Annealing curves
**HIGH** fluorescence
and fluorescence transition at
$T_m$ observed
**LOW** fluorescence
**NO** fluorescence transition at
$T_m$ observed

Figure 12

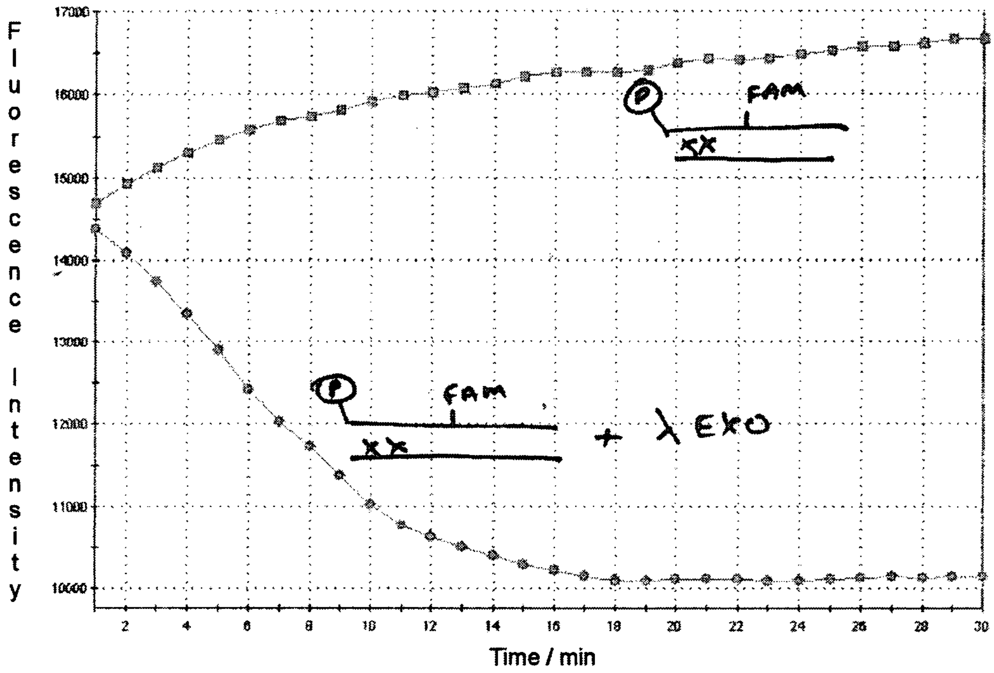


Figure 13

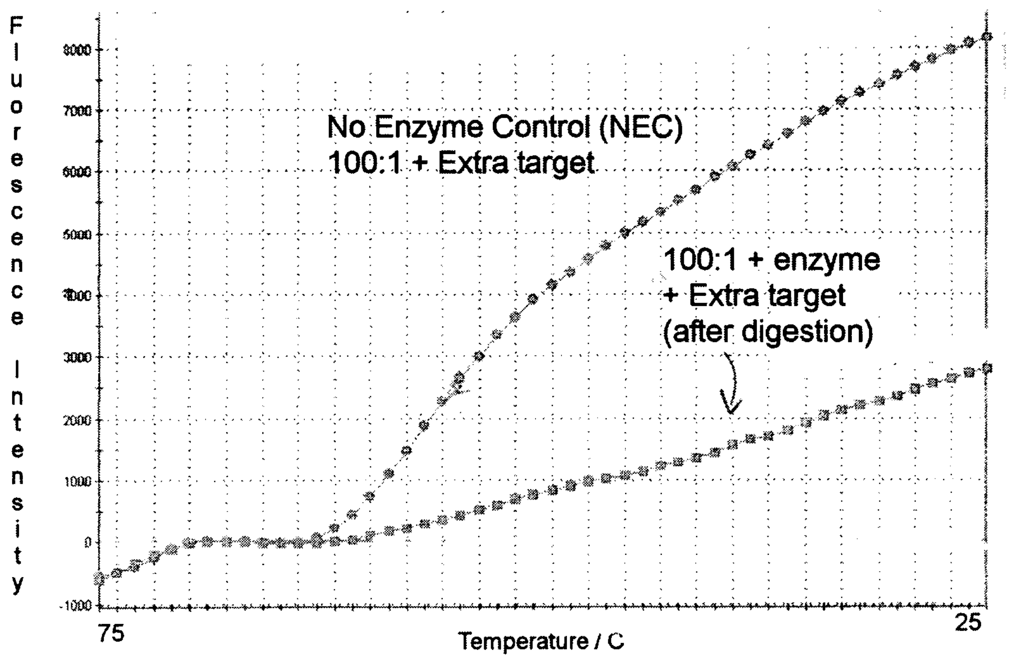

Figure 14

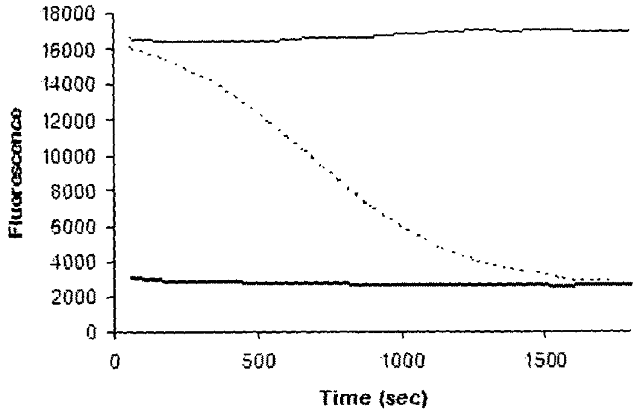


Figure 15

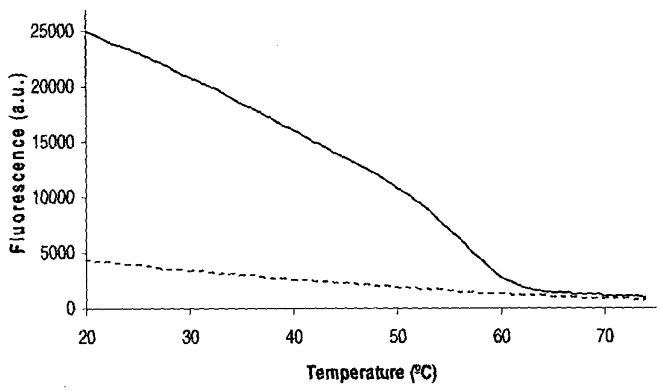

Figure 16

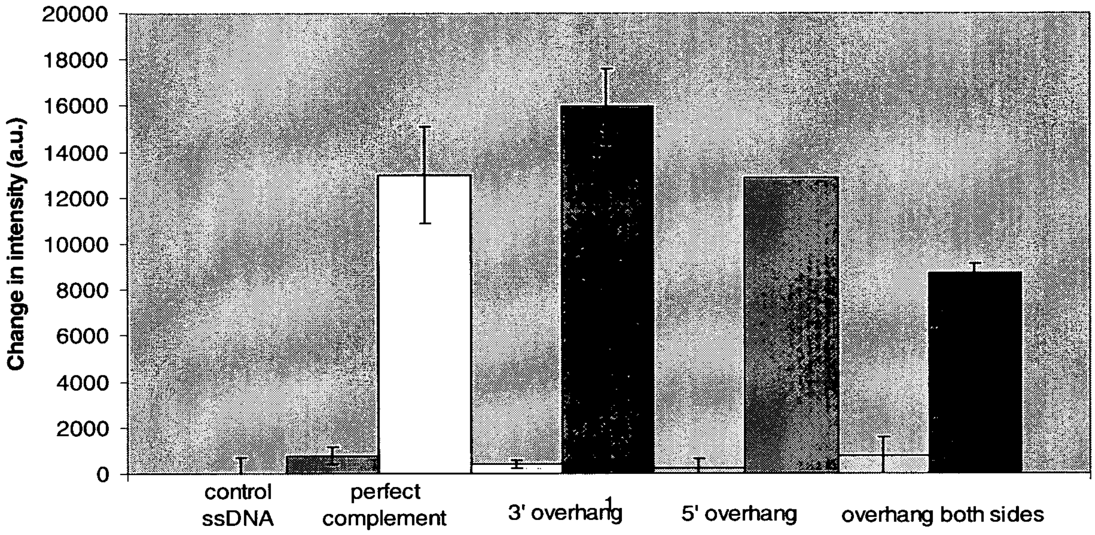


Figure 17

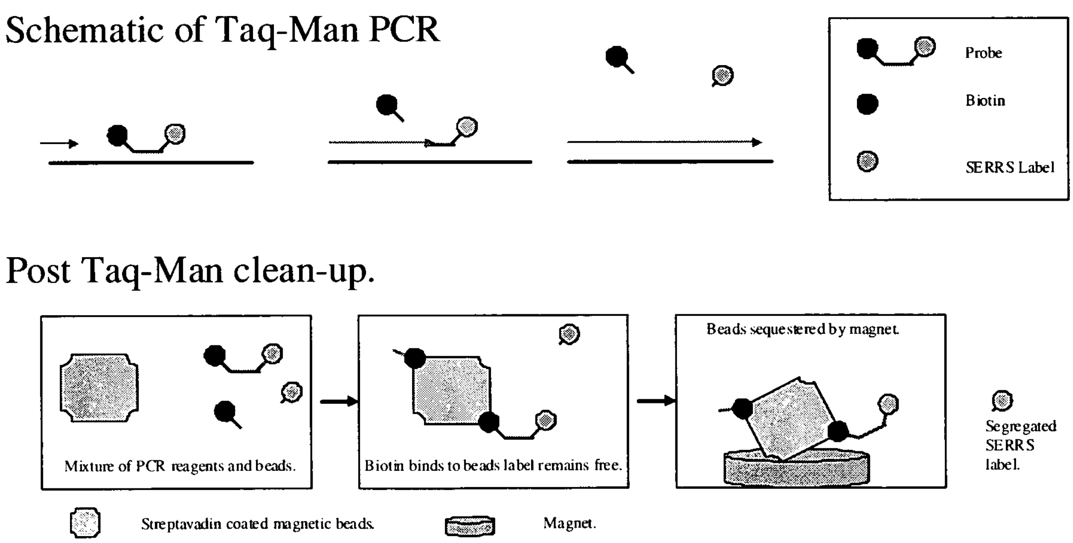

Figure 18

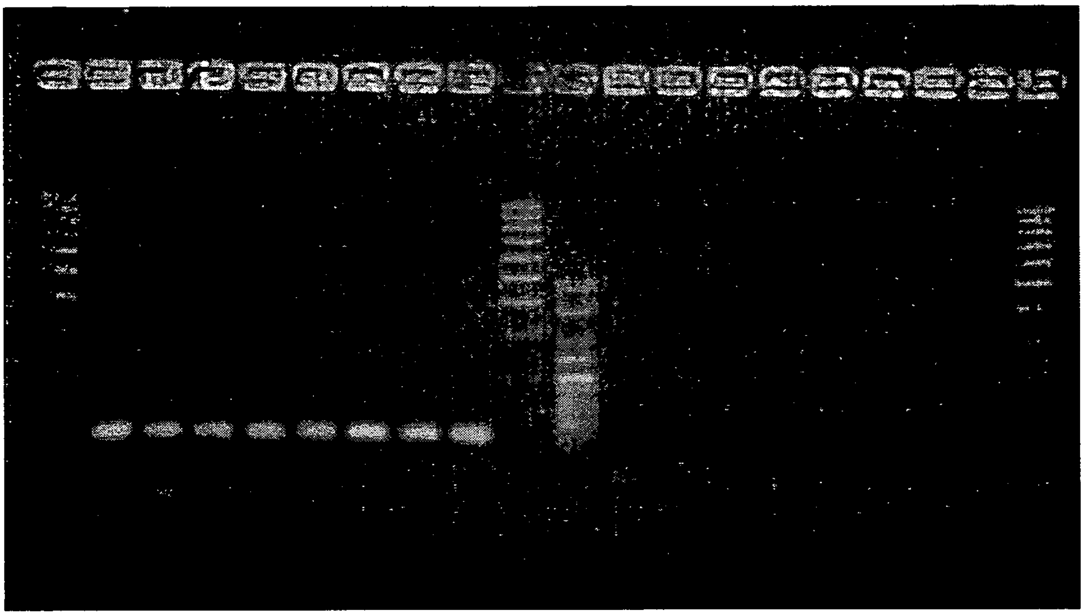

Figure 19

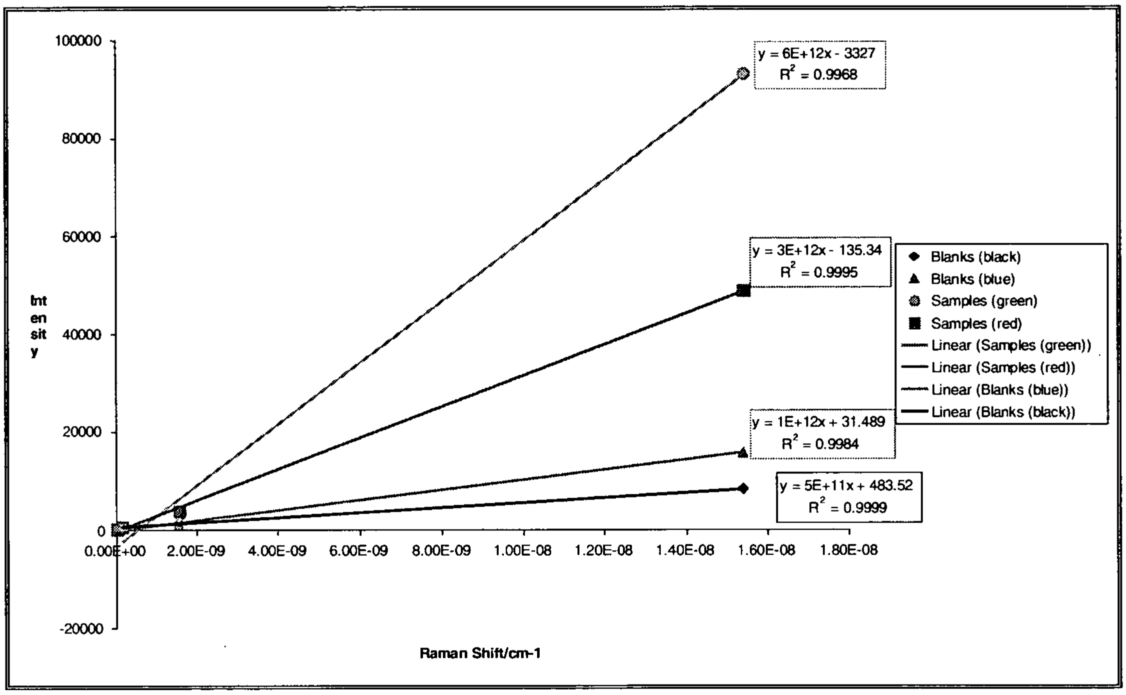

Figure 20

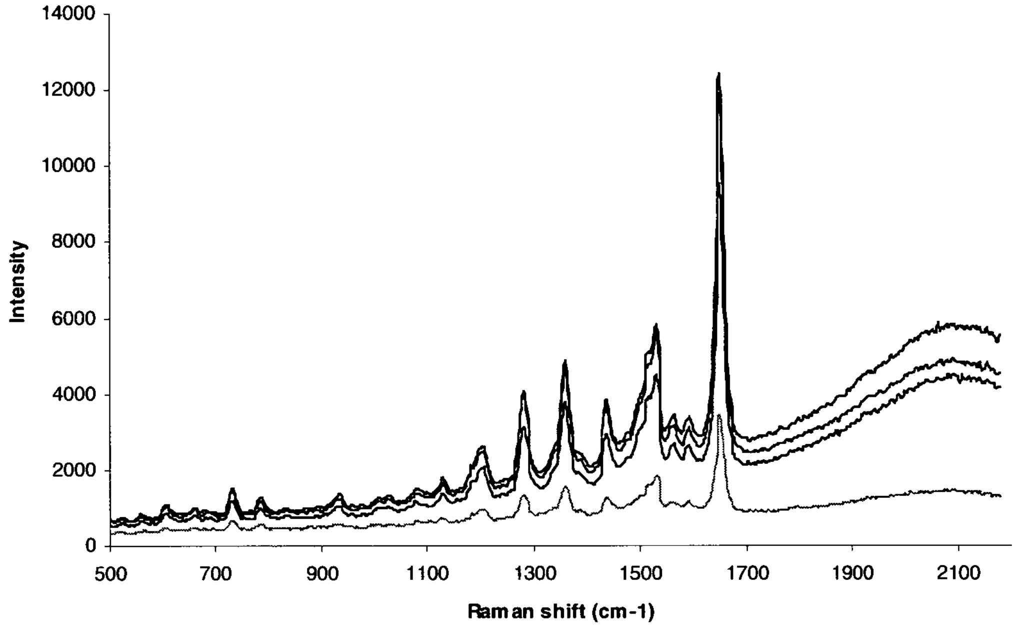


Figure 21

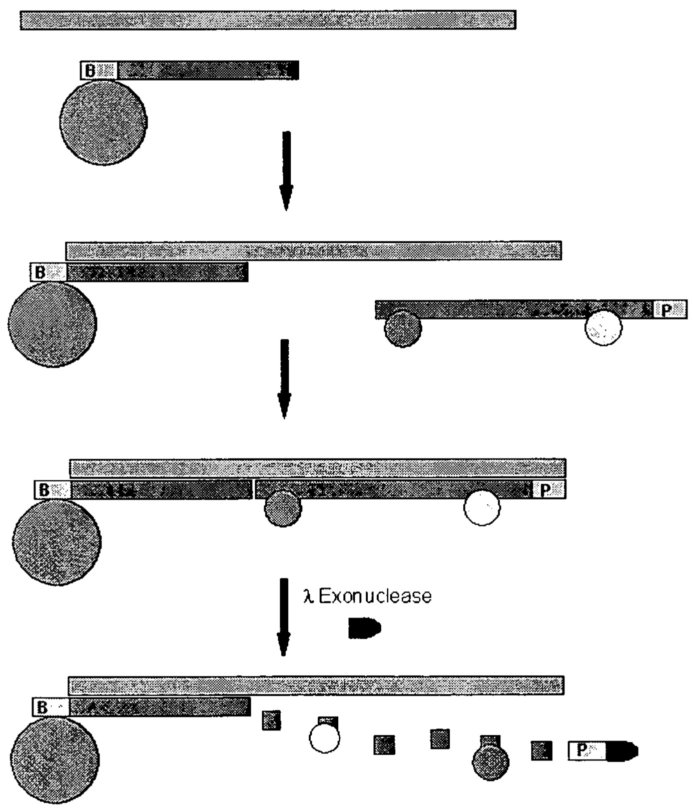

Figure 22

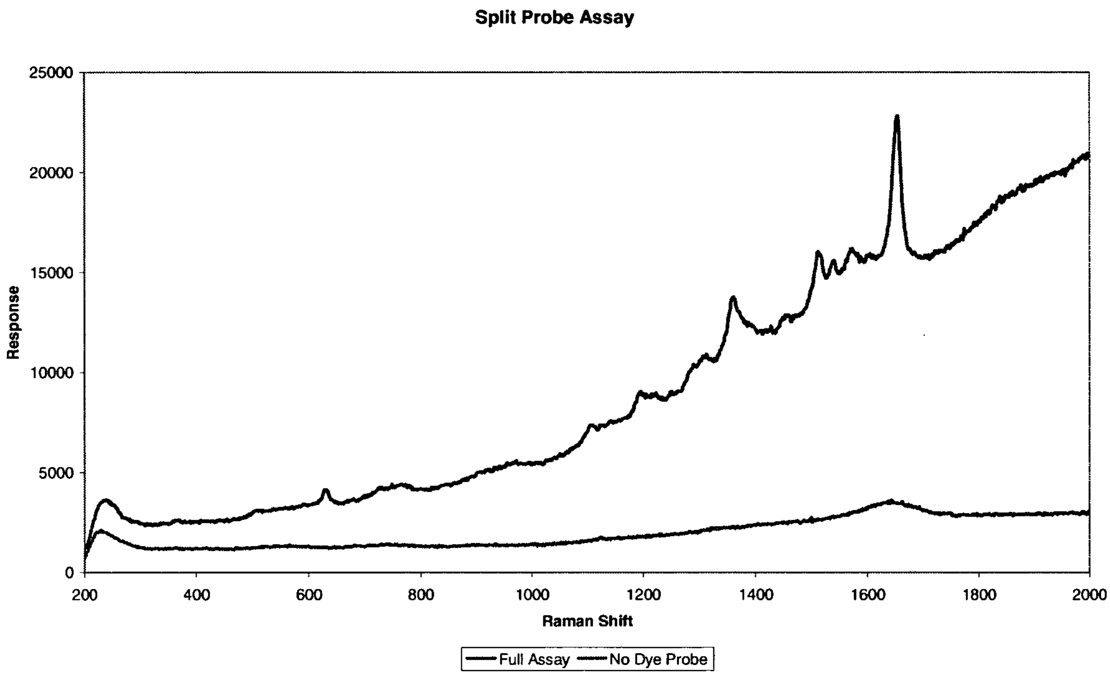
Split Probe Assay
25000
20000
15000
10000
5000
0
Response
200
400
600
800
1000
1200
1400
1600
1800
2000
Raman Shift
Full Assay
No Dye Probe

Figure 23

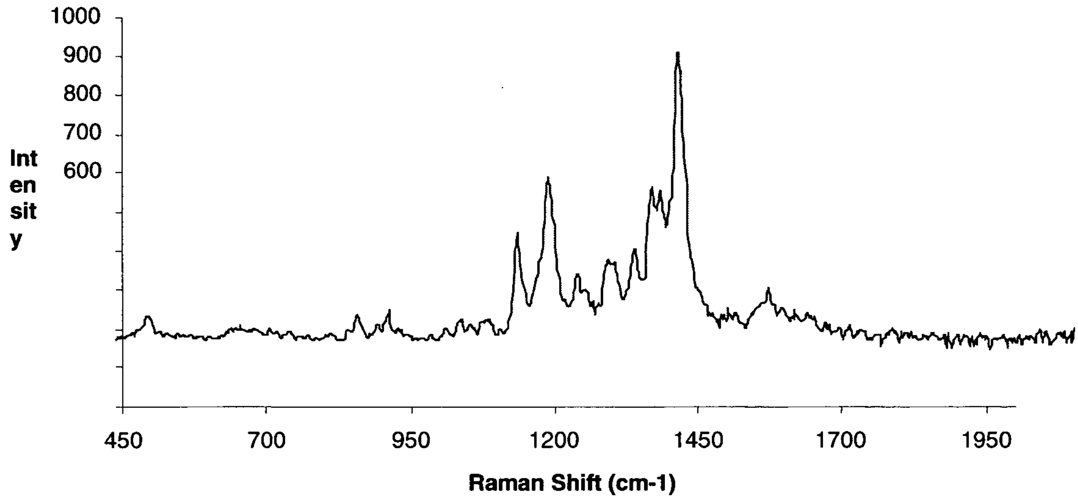


Figure 24

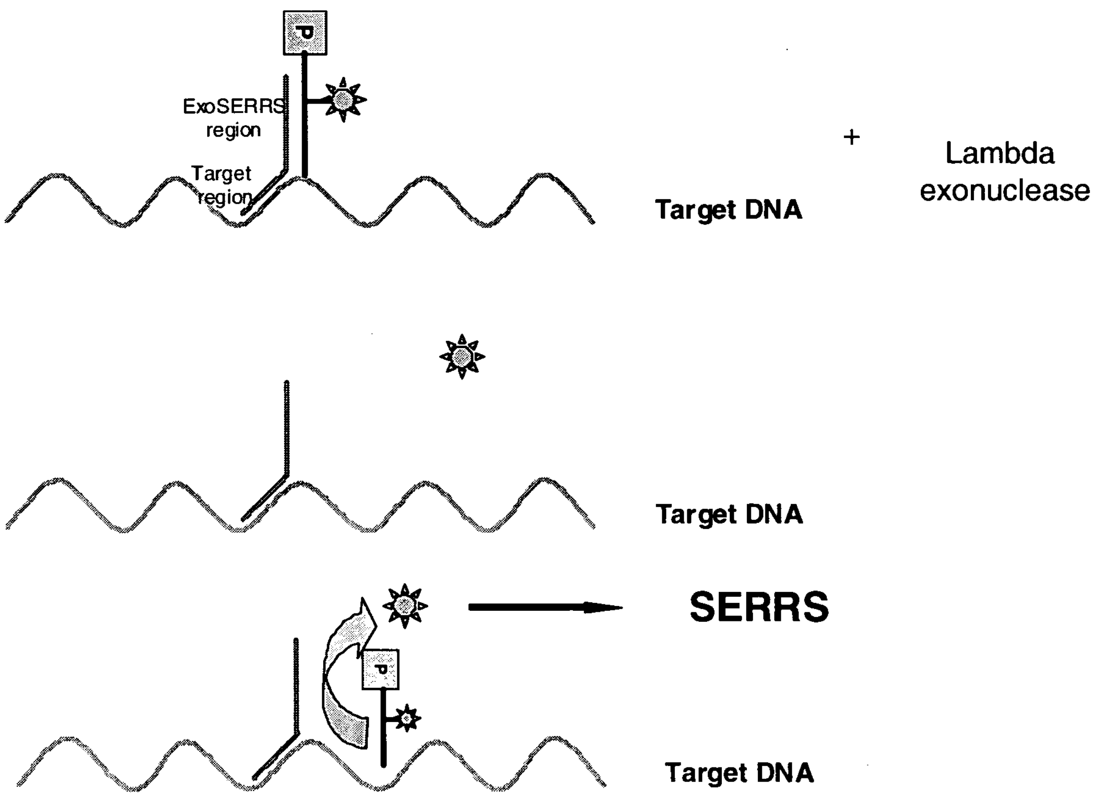

Figure 25

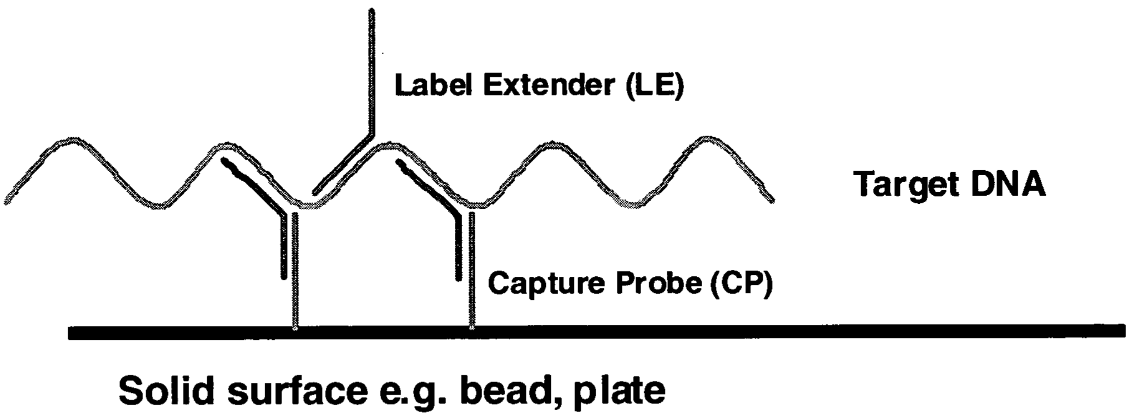
Label Extender (LE)
Target DNA
Capture Probe (CP)
Solid surface e.g. bead, plate

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5853990 A, Winger
- WO 9705280 A
- WO 9960157 A
- WO 2005019812 A


### Non-patent literature cited in the description


- **S.C. HILLIER et al.** *Electrochemistry Communications,* 2004, vol. 6, 1227-1232
- **MCHUGH, C.J. ; DOCHERTY, F.T. ; GRAHAM, D. ; SMITH, W.E.** *Analyst,* 2004, vol. 129 (1), 69-72
- **GRAHAM, D ; FAULDS, K ; SMITH, W. E.** *Chemical Communications,* 2006, vol. 42, 4363-4371
- **SAMBROOK et al.** Molecular Cloning: A Laboratory Manual. Cold Spring Harbor Laboratory Press, 2001, 7.9-7.12
- **J SAMBROOK et al.** Molecular Cloning-A Laboratory Manual. Cold Spring Harbor
- **BOLTON ; MCCARTHY.** *P.N.A.S.,* 1962, vol. 48, 1390
- **MCHUGH, C.J. ; DOCHERTY, F.T. ; GRAHAM, D. ; SMITH, W.E.** *Analyst,* vol. 129 (1), 69-72
- **G. TOLUN ; S. MYERS.** A real-time DNase assay (ReDA) based on PicoGreen® fluorescence. *Nucleic Acids Res.,* 2003, vol. 31, e111
- **P.G. MITSIS ; J.G. KWAGH.** *Nucleic Acids Research,* 1999, vol. 27 (15), 3057-3063
- **J. J. STORHOFF et al.** *Nature Biotech.,* 2004, vol. 22 (7), 883-887
- **MOORE et al.** *Nature Biotech.,* 2004, vol. 22, 1133-1138
- **X. DOU ; Y. M. JUNG ; Z. CAO ; Y. OZAKI.** *Appl. Spectrosc.,* 1999, vol. 53, 1140
- **M. TENG ; N. USMAN ; C.A. FREDERICK ; H.J. WANG.** *Nucl. Acids Res.,* 1988, vol. 16 (6
- **D.E. WEMER.** *Biopolymers,* 1999, vol. 52, 197-211
- **F.M. HO ; E.A.H HALL.** *Biosensors and Bioelectronics,* 2004, vol. 20 (5), 1001-1010
- **F.M. CAMPBELL et al.** *Analyst,* 2008

SEQUENCE LISTING

<110> The University of Strathclyde
Graham, Duncan
Faulds, Karen
Smith, Ewen
Ricketts, Alastair

<120> IDENTIFICATION OF NUCLEIC ACID SEQUENCES

<130> P15146PC

<160> 27

<170> PatentIn version 3.3

<210> 1
<211> 18
<212> DNA
<213> Artificial

<220>
<223> oligonucleotide probe

<400> 1
ttttcccagt cacgacgt 18

<210> 2
<211> 18
<212> DNA
<213> Artificial

<220>
<223> oligonucleotide probe

<400> 2
ttttcccagt cacgacgt 18

<210> 3
<211> 18
<212> DNA
<213> Artificial

<220>
<223> oligonucleotide probe

<400> 3
ttttcccagt cacgacgt 18

<210> 4
<211> 18
<212> DNA
<213> Artificial

<220>
<223> Oligonucleotide probe

<220>
<221> modified_base
<222> (10)..(10)
<223> Fluorescein dT

<220>
<221> misc_feature
<222> (10)..(10)
<223> n is a, c, g, or t

<400> 4
ttttcccagn cacgacgt 18

<210> 5
<211> 18

<212> DNA
<213> Artificial

<220>
<223> Oligonucleotide probe

<400> 5
acgtcgtgac tgggaaaa 18

<210> 6
<211> 38
<212> DNA
<213> Artificial

<220>
<223> oligonucelotide probe

<400> 6
acgtcgtgac tgggaaaacc ctggcgttac ccaactta 38

<210> 7
<211> 38
<212> DNA
<213> Artificial

<220>
<223> oligonucleotide probe

<400> 7
tcactggccg tcgttttaca acgtcgtgac tgggaaaa 38

<210> 8
<211> 54
<212> DNA
<213> Artificial

<220>
<223> oligonucleotide probe

<400> 8
tcactggccg tcgttttaca acgtcgtgac tgggaaaacc ctggcgttac ccaa 54

<210> 9
<211> 18
<212> DNA
<213> Artificial

<220>
<223> oligonucleotide probe

<220>
<221> modified_base
<222> (10)..(10)
<223> dT TAMRA

<220>
<221> misc_feature
<222> (10)..(10)

<223> n is a, c, g, or t

<400> 9
ttttcccagn cacgacgt 18

<210> 10
<211> 18
<212> DNA
<213> Artificial

<220>
<223> oligonucleotide probe

<220>
<221> modified_base
<222> (10)..(10)
<223> dT FAM

<400> 10
ttttcccagt cacgacgt 18

<210> 11
<211> 18
<212> DNA
<213> Artificial

<220>
<223> oligonucleotide probe

<400> 11
acgtcgtgac tgggaaaa 18

<210> 12
<211> 22
<212> DNA
<213> Artificial

<220>
<223> oligonucleotide probe

<220>
<221> modified_base
<222> (12)..(12)
<223> dT FAM

<220>
<221> misc_feature
<222> (12)..(12)
<223> n is a, c, g, or t

<400> 12
gctaaacttg cntgccactc at 22

<210> 13
<211> 22
<212> DNA
<213> Artificial

<220>
<223> Oligonucleotide probe

<400> 13
atgagtggca agcaagttta gc 22

<210> 14
<211> 100
<212> DNA
<213> Artificial

<220>
<223> oligonucleotide probe

<400> 14

ggaatttcca cttgatatta ccgcaggaac agaagctgcg acagggacta aggatgcctc 60

tattgactac catgagtggc aagcaagttt agccctttct 100

<210> 15
<211> 18
<212> DNA
<213> Artificial

<220>
<223> oligonucleotide probe

<220>
<221> modified_base
<222> (10)..(10)
<223> dT FAM

<220>
<221> misc_feature
<222> (10)..(10)
<223> n is a, c, g, or t

<400> 15
ttttcccagn cacgacgt 18

<210> 16
<211> 18
<212> DNA
<213> Artificial

<220>
<223> oligonucleotide probe

<220>
<221> modified_base
<222> (10)..(10)
<223> dT TAMRA

<220>
<221> misc_feature
<222> (10)..(10)
<223> n is a, c, g, or t

<400> 16
ttttcccagn cacgacgt 18

<210> 17
<211> 18
<212> DNA
<213> Artificial

<220>
<223> Oligonucleotide probe

<400> 17
ttttcccagt cacgacgt 18

<210> 18
<211> 18
<212> DNA
<213> Artificial

<220>
<223> oligonucleotide probe

<400> 18
acgtcgtcac tgcgaaaa 18

<210> 19
<211> 38
<212> DNA
<213> Artificial

<220>
<223> oligonucleotide probe

<400> 19
acgtcgtcac tgcgaaaacc ctggcgttac ccaactta 38

<210> 20
<211> 38
<212> DNA
<213> Artificial

<220>
<223> oligonucleotide probe

<400> 20
tcactggccg tcgttttaca acgtcgtcac tgccgaaa 38

<210> 21
<211> 58
<212> DNA
<213> Artificial

<220>
<223> oligonucleotide probe

<400> 21
tcactggccg tcgttttaca acgtcgtcac tgccgaaacc ctggcgttac ccaactta 58

<210> 22
<211> 24
<212> DNA
<213> Artificial

<220>
<223> Primer

<400> 22
cacttratat taccgcagga acag 24

<210> 23
<211> 21
<212> DNA
<213> Artificial

<220>
<223> Primer

<400> 23
gctaaacttg ctgccactca t 21

<210> 24
<211> 27
<212> DNA
<213> Artificial

<220>
<223> oligonucleotide probe

<400> 24
agaggcatcc ttagtccctg tcgcagc 27

<210> 25
<211> 12
<212> DNA
<213> Artificial

<220>
<223> oligonucleotide probe

<220>
<221> modified_base
<222> (1)..(1)
<223> dT biotinylated

<220>
<221> misc_feature
<222> (1)..(1)
<223> n is a, c, g, or t

<400> 25
nctccgtagg aa 12

<210> 26
<211> 15
<212> DNA
<213> Artificial

<220>
<223> oligonucleotide probe

<220>
<221> modified_base
<222> (1)..(1)
<223> dT modified with BHQ2

<220>
<221> misc_feature
<222> (1)..(1)
<223> n is a, c, g, or t

<220>
<221> modified_base
<222> (13)..(13)
<223> dT TAMRA

<220>
<221> misc_feature
<222> (13)..(13)
<223> n is a, c, g, or t

<400> 26
ncagggacag cgncg 15

<210> 27
<211> 27
<212> DNA
<213> Artificial

<220>
<223> oligonucleotide

<400> 27
agaggcatcc ttagtccctg tcgcagc 27